# DEMANDE DE BREVET EUROPEEN

(11) **EP 1 864 649 A2**
(43) Date de publication de la demande: **12.12.2007**
(21) Numéro de dépôt: 07106603.9
(22) Date de dépôt: 20.04.2007
(51) Int. Cl.: A61K 8/898, A61Q 5/06, C08G 77/44

(54) **Composé de type bis-urée, composition le comprenant, utilisation et procédé de traitement cosmétique**

(30) Priorité: 09.05.2006 FR 0651653
(71) Demandeur: L'Oréal, 75008 Paris (FR)
(72) Inventeur: Chodorowski-Kimmes, Sandrine, 60300, SENLIS (FR)
(74) Mandataire: Dodin, Catherine

(57) **Abrégé**

La présente invention concerne une composition cosmétique ou pharmaceutique comprenant, dans un milieu physiologiquement acceptable, une phase grasse et au moins un composé de formule générale (I), employé pour texturer ladite phase grasse:

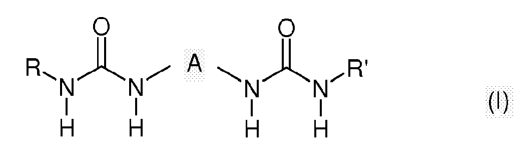

L'invention concerne également l'utilisation d'un tel composé pour texturer ladite composition, ainsi que lesdits composés de formule générale (I).

L'invention concerne encore un procédé de traitement cosmétique employant ladite composition.

## Description

La présente invention a trait à des compositions notamment cosmétiques ou pharmaceutiques à phase grasse liquide texturée par un composé de type bis-urée.

Pour structurer les huiles et leur donner la texture ou la viscosité souhaitée, l'utilisation d'organogélateurs est bien connue de l'homme de l'art. Les organogélateurs sont connus comme étant de petites molécules capables de structurer un milieu organique, à faible concentration. Ils peuvent modifier les interactions moléculaires à l'intérieur de l'huile et changer ses caractéristiques physiques et/ou chimiques. Toutefois, la solubilisation de ces molécules organogélatrices dans une huile ou un mélange d'huiles requiert une température souvent élevée, ce qui peut générer des coûts supplémentaires de chauffage et surtout être incompatible avec la présence de molécules thermosensibles. De plus, le gel ainsi obtenu ne présente pas toujours la stabilité nécessaire au cours du temps : l'organogélateur peut précipiter et/ou exsuder au cours du temps.

Plusieurs classes d'organogélateurs sont bien connues de l'homme de l'art, dont les composés de type bis-urées.
L'utilisation de certaines bis-urées à titre d'organogélateur a été envisagée notamment dans les documents WO02/47628, JP 2003-064346, JP 08-237942 et JP10-236981. Ces documents décrivent entre autre l'utilisation de bis-urées pour texturer des milieux, cosmétiques ou non.
Il existe aussi de nombreux articles décrivant des molécules organiques fonctionnalisées par une ou plusieurs urées dont notamment les articles de Bouteiller et al., dans New J.Chem., 2000, 24, 845-848; Langmuir, 2002, 18, 7218-7222 et J.Am.Chem.Soc. 2003, 125, 13148-13154 décrivant l'utilisation de certaines bis-urées dans des solvants organiques tel que le toluène, le tétrachlorure de carbone ou le dodécane, à des fins de gélification de ces derniers.
On peut aussi citer Hanabusa et coll. qui décrit le comportement de molécules bis-urées comme organogélateur (Langmuir 2003, 19(21), 8622-8624).
L'article de Hamilton et al. dans Tetrahedron Letters, 1998, 39, 7447-7450 décrit l'aptitude de certains dérivés bis-urées à se comporter comme des gélifiants dans certains solvants organiques.
Toutefois, toutes les bis-urées décrites dans ces documents ne se solubilisent pas à température ambiante et/ou dans l'ensemble des huiles cosmétiques, et notamment dans les huiles siliconées, seules ou en mélange avec des huiles carbonées.

Dans le domaine de la texturisation de compositions comprenant des huiles ou solvants siliconés, on peut citer notamment JP2004262856 qui décrit des dérivés siliconés d'acides aminés, notamment des dérivés de valine substitués par des chaînes PDMS linéaires ou greffées, capables de gélifier les huiles de silicone, sans être limité par les autres composants de la formule.
On peut également citer JP2004182692 et JP2004182693 qui décrivent comme gélateur des huiles siliconées, des dérivés de bis-amides siliconés qui peuvent être cycliques ou non.
Toujours en cosmétique, on peut encore citer W09736572 qui décrit une composition cosmétique formée d'une huile comportant au moins un motif silicone et au moins un agent gélifiant composé de motif siloxane et d'un groupement formant des liaisons hydrogène.
On peut encore citer WO03/105801 qui décrit une composition cosmétique comprenant une phase grasse au moins partiellement siliconée et structurée par une PDMS comportant au moins deux groupements capable de former une liaison H.
On peut aussi citer W02004/052963 qui décrit des polymères siloxanes comprenant des groupes capables de former au moins 4 liaisons hydrogène, et susceptibles de gélifier des compositions notamment cosmétiques.
Enfin, on peut noter W02005/005557 qui cite notamment l'utilisation comme agent de contrôle en rhéologie, d'un agent issu de la réaction entre un ou plusieurs polyisocyanates et une ou plusieurs amines. L'amine peut comporter un hétéroatome.
Dans les domaines non cosmétiques, on peut mentionner EP0406731 qui décrit des molécules bis-urées monofonctionnalisées par des fonctions siloxane. Ce type de molécules est connu dans le domaine de l'adhésion, le traitement de surfaces et celui des textiles, notamment.

Toutefois, aucun de ces documents ne propose de famille chimique comprenant des composés susceptibles de texturer, d'épaissir, voire de gélifier, des milieux cosmétiques variés, tels que des milieux constitués uniquement d'huiles et/ou de solvants carbonés ou hydrocarbonés, ou bien constitués uniquement d'huiles siliconées, ou encore constitués d'un mélange d'huiles carbonées et siliconées.

Ainsi, la demanderesse est à la recherche de tels composés, capables de texturer et/ou structurer les milieux cosmétiques non aqueux, qu'ils comprennent des huiles cosmétiques siliconées et/ou non siliconées (carbonées).
On sait que, pour identifier un composé gélifiant capable de texturer de manière satisfaisante une composition cosmétique particulière et apte à être solubilisé à température ambiante dans les huiles de ladite composition, de nombreux tests doivent généralement être réalisés au préalable.
Il existe donc un besoin de composés texturants (ou organogélateurs) que l'on pourrait qualifier d'universels dans la mesure où ils seraient efficaces pour texturer, à température ambiante, un grand nombre d'huiles cosmétiques siliconées et non siliconées, ainsi que leur mélange.

La présente invention a précisément pour but de proposer de nouveaux composés susceptibles de texturer, épaissir, voire gélifier, les compositions cosmétiques, quelle que soit la nature des huiles qu'elles comprennent.

Un objet de la présente invention est donc une composition cosmétique ou pharmaceutique comprenant, dans un milieu physiologiquement acceptable, une phase grasse et au moins un composé de formule générale (1) telle que définie ci-après, ou l'un de ses sels et/ou isomères.
Un autre objet de l'invention est l'utilisation d'un tel composé de formule (1) pour texturer une composition cosmétique ou pharmaceutique comprenant, dans un milieu physiologiquement acceptable, une phase grasse.
Un autre objet de l'invention consiste en certains composés de formule générale (la), ou l'un de leurs sels et/ou isomères.

De manière inattendue, on a découvert que les composés de bis-urée tels que définis ci-dessus, ainsi que leurs mélanges, étaient des agents texturants, notamment épaississants, voire gélifiants, de choix pour répondre au besoin en organogélateurs universels exprimés plus haut; en particulier, les composés selon l'invention se solubilisent à température ambiante dans une large gamme d'huiles et de solvants cosmétiques, carbonés et/ou siliconés, et peuvent permettre de modifier la viscosité de ces huiles et solvants même à de faibles concentrations, notamment inférieures à 5% en poids.

Les composés de type bis-urée selon l'invention répondent à la formule générale (1) suivante : 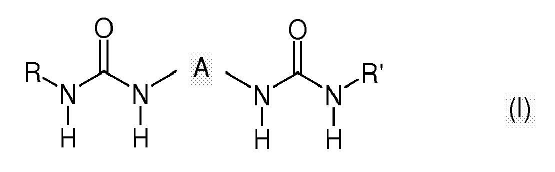 dans laquelle :
- A est un groupement de formule (II): 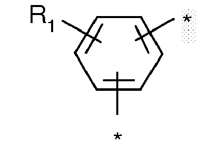 avec R1 étant un radical alkyle C₁ à C₄ linéaire ou ramifié, et les * symbolisant les points de rattachement du groupement A à chacun des deux atomes d'azote du reste du composé de formule générale (1), et
- R et R', identiques ou différents, sont choisis parmi :
   - i) les radicaux de formule (III) : 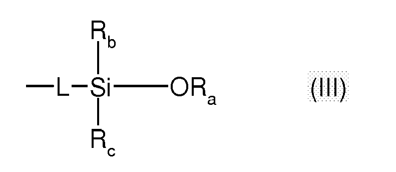
   dans laquelle :
   - L est une liaison simple ou un radical divalent carboné, notamment hydrocarboné (alkylène), linéaire, ramifié et/ou cyclique, saturé ou insaturé, comprenant 1 à 18 atomes de carbone, et pouvant comprendre 1 à 4 hétéroatomes choisis parmi N, O et S;
   - Ra est :
      a) un radical carboné, notamment hydrocarboné (alkyle), linéaire, ramifié et/ou cyclique, saturé ou insaturé, comprenant 1 à 18 atomes de carbone, et pouvant comprendre 1 à 8 hétéroatomes choisis parmi N, O, Si et S; ou bien
      b) un radical siliconé de formule : 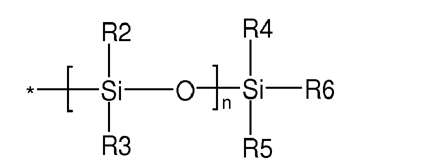 avec n étant compris entre 0 et 100, notamment entre 1 et 50, voire 2 à 30, ou encore 3 à 20;
         et R2 à R6 étant, indépendamment les uns des autres, des radicaux carbonés, notamment hydrocarbonés (alkyles) linéaires ou ramifiés, ayant 1 à 12, notamment 1 à 6 atomes de carbone, et pouvant comprendre 1 à 4 hétéroatomes, notamment O;
   - Rb et Rc sont, indépendamment l'un de l'autre, choisis parmi:
      a) les radicaux carbonés, notamment hydrocarbonés (alkyles), linéaires, ramifiés et/ou cycliques, saturés ou insaturés, comprenant 1 à 18 atomes de carbone, et pouvant comprendre 1 à 4 hétéroatomes choisis parmi N, O, Si et S;
      b) les radicaux de formule : 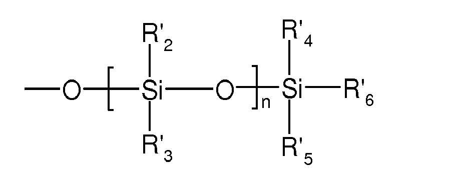 avec n étant compris entre 0 et 100, notamment entre 1 et 50, voire 2 à 30, ou encore 3 à 20;
         et R'2 à R'6 étant, indépendamment les uns des autres, des radicaux carbonés, notamment hydrocarbonés (alkyles) linéaires ou ramifiés, ayant 1 à 12, notamment 1 à 6 atomes de carbone, et pouvant comprendre 1 à 4 hétéroatomes, notamment O.
         et
   - ii) les radicaux alkyle en C₁ à C₃₀, linéaire, ramifié et/ou cyclique, saturé ou insaturé, et comprenant éventuellement 1 à 3 hétéroatomes choisis parmi O, S, FetN;
   étant entendu que l'un au moins des radicaux R et/ou R' est de formule (III).

On a en effet constaté que cette famille de composés bis-urée pouvait permettre la texturation de milieux siliconés, notamment lorsque les deux radicaux R et R' étaient siliconés, c'est-à-dire de formule (III).
Lorsque l'un des radicaux R ou R' est non siliconé, c'est-à-dire est un radical alkyle tel que défini plus haut, il est possible de texturer avec ces composés les milieux siliconés, mais aussi les milieux carbonés et les milieux comprenant un mélange d'huiles siliconées et carbonées.

Notamment le groupement A peut être de formule : 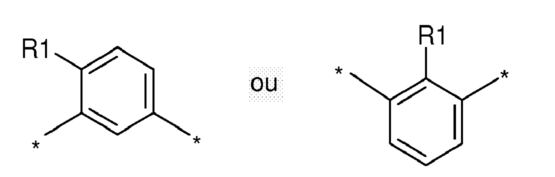 avec R1 et les * étant tels que définis précédemment.

En particulier, R1 peut être un groupement méthyle, ce qui conduit à un groupement A de formule : 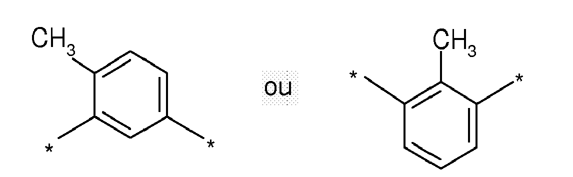 dans laquelle les * sont tels que définis précédemment.
En particulier, les composés selon l'invention peuvent être sous forme de mélange lié au fait que A peut être un mélange de 2,4-tolylène et 2,6-tolylène, notamment dans des proportions (isomère 2,4)/(isomère 2,6) variant de 95/5 à 80/20.

Selon l'invention, l'un au moins des radicaux R et/ou R' doit être de formule (III): 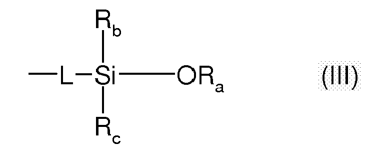 Dans cette formule, L est de préférence un radical divalent carboné, notamment hydrocarboné (alkylène), linéaire, ramifié et/ou cyclique, saturé ou insaturé, comprenant 1 à 18 atomes de carbone, et pouvant comprendre 1 à 4 hétéroatomes choisis parmi N, O et S. Dans le radical L, la chaîne carbonée peut être interrompue par le/les hétéroatomes et/ou peut comprendre un substituant comprenant ledit/lesdits hétéroatomes.
En particulier, L peut être de structure -(CH₂)n- avec n= 1 à 18, notamment 2 à 12, voire 3 à 8. De préférence, L est choisi parmi les radicaux méthylène, éthylène, propylène, butylène et notamment n-butylène ou octylène.
Le radical L peut également être ramifié, par exemple du type -CH₂-CH(CH₃)-, ce qui conduit au radical de formule (III) suivant :

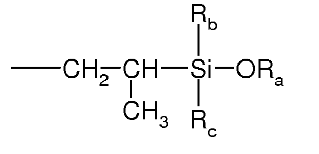

Le radical Ra peut être un radical carboné, notamment hydrocarboné (alkyle), linéaire, ramifié et/ou cyclique, saturé ou insaturé, comprenant 1 à 18 atomes de carbone, et pouvant comprendre 1 à 8 hétéroatomes choisis parmi N, O, Si et S. La chaîne carbonée peut être interrompue par le/les hétéroatomes et/ou peut comprendre un substituant comprenant ledit/lesdits hétéroatomes; les hétéroatomes peuvent notamment former un ou plusieurs groupements -SiO- (ou -OSi)-.

Ainsi, le radical Ra peut être de structure -(CH₂)n'-CH₃ avec n'= 0 à 17, notamment 1 à 12, voire de 1 à 6. En particulier, Ra peut être méthyle, éthyle, propyle ou butyle.
Il peut également être de structure -(CH₂)x-O-(CH₂)z-CH₃ ou bien -(CH₂)x-O-(CH₂)y-O-(CH₂)z-CH₃, avec x = 1 à 10, de préférence 2; y=1 à 10 de préférence 2, et z= 0 à 10, de préférence 0 ou 1.
Le radical Ra peut encore être de structure -SiR₄R₅R₆ (cas où n=0), dans laquelle R4, R5 et R6 sont, indépendamment les uns des autres, de préférence, des radicaux alkyle, ayant 1 à 12 atomes de carbone, notamment 1 à 6 atomes de carbone; en particulier R4, R5 et/ou R6 peuvent être choisis parmi méthyle, éthyle, propyle, butyle.
Le radical Ra peut également être un radical siliconé de formule: 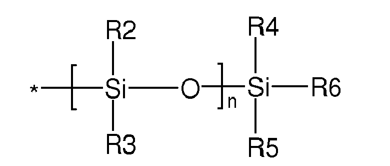 dans laquelle R2 à R6 sont, indépendamment les uns des autres, de préférence, des radicaux alkyle, ayant 1 à 12 atomes de carbone, notamment 1 à 6 atomes de carbone; en particulier R2 à R6 peuvent être choisis parmi méthyle, éthyle, propyle, butyle;
et en particulier un radical : 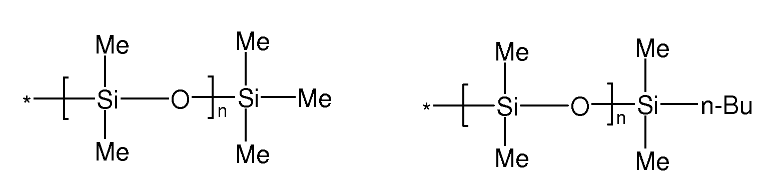 avec n=1 à 100; et encore plus particulièrement un radical :

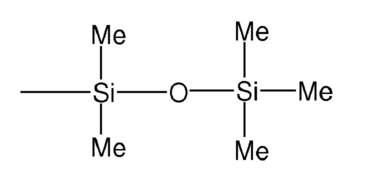

Les radicaux Rb et Rc, identiques ou différents, peuvent être des radicaux carbonés, notamment hydrocarbonés (alkyles), linéaires, ramifiés et/ou cycliques, saturés ou insaturés, comprenant 1 à 18 atomes de carbone, et pouvant comprendre 1 à 8 hétéroatomes choisis parmi N, O, Si et S. Dans ces radicaux, la chaîne carbonée peut être interrompue par le/les hétéroatomes et/ou peut comprendre un substituant comprenant ledit/lesdits hétéroatomes; les hétéroatomes peuvent notamment former un ou plusieurs groupements -SiO- (ou - OSi)- .
Ainsi, ils peuvent être de structure -(CH₂)m-CH₃ avec m= 0 à 17, notamment 1 à 12, voire 2 à 5; en particulier, Rb et/ou Rc peuvent être méthyl, éthyle, propyle ou butyle;
Ils peuvent également être de structure -O-(CH₂)m'-CH₃ avec m'= 0 à 5, notamment 1 à 4, et en particulier méthoxy ou éthoxy.
Ils peuvent aussi être de structure -O-(CH₂)x-O-(CH₂)z-CH₃ ou -O-(CH₂)x-O-(CH₂)y-O-(CH₂)z-CH₃, avec x = 1 à 10, de préférence 2; y=1 à 10 de préférence 2, et z= 0 à 10, de préférence 0 ou 1.
Ils peuvent encore être de structure : 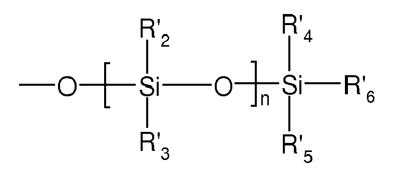 avec n étant compris entre 0 et 100, notamment entre 1 et 50, voire 2 à 30, ou encore 3 à 20;
et R'2 à R'6 étant, indépendamment les uns des autres, de préférence, des radicaux alkyle, ayant 1 à 12 atomes de carbone, notamment 1 à 6 atomes de carbone; en particulier R'2 à R'6 peuvent être choisis parmi méthyle, éthyle, propyle, butyle.

Lorsqu'ils sont de formule (III), les radicaux R et/ou R' sont de préférence choisis parmi les radicaux suivants : 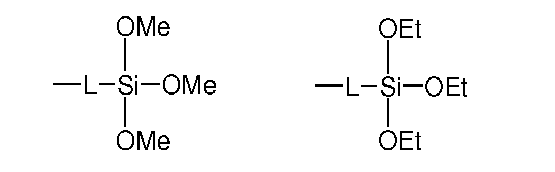 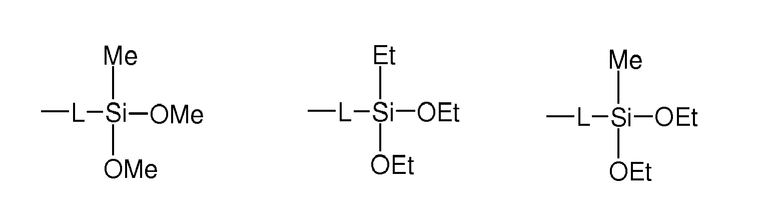 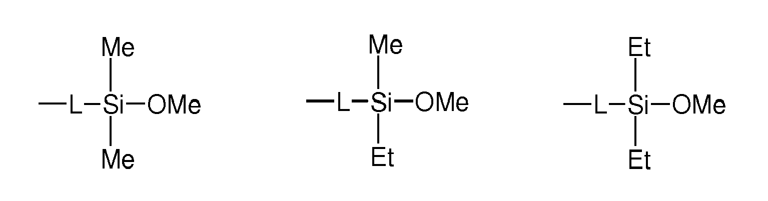 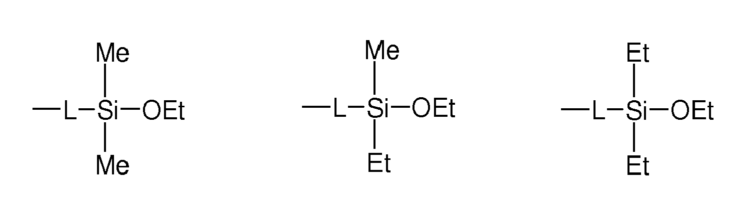 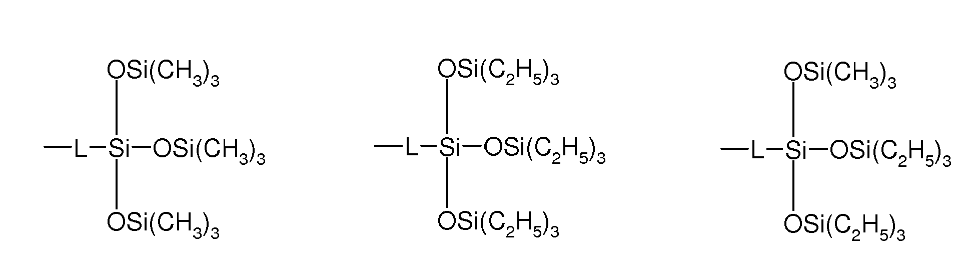 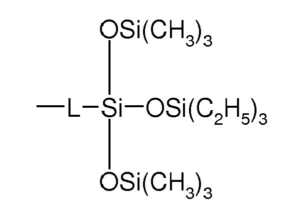 et également ceux de formule : 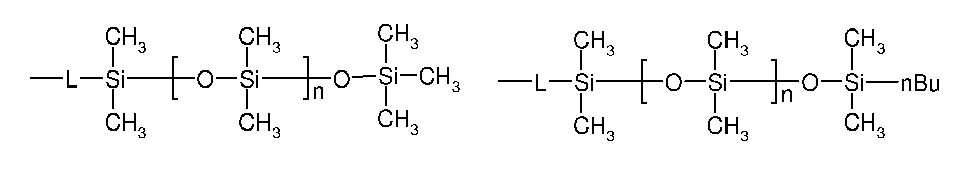 avec n variant de 0 à 100 et en particulier 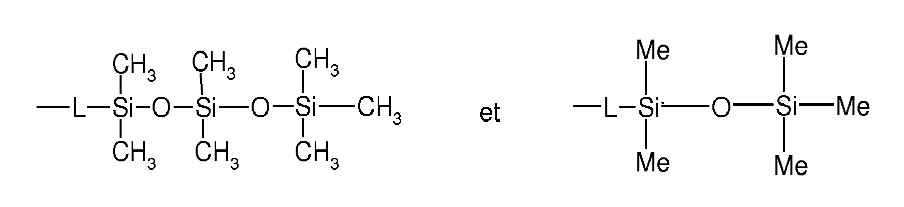 et 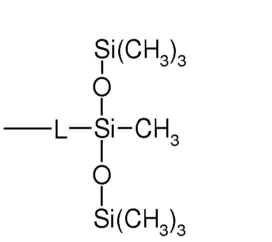 ou encore 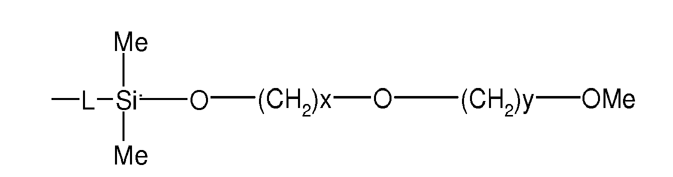 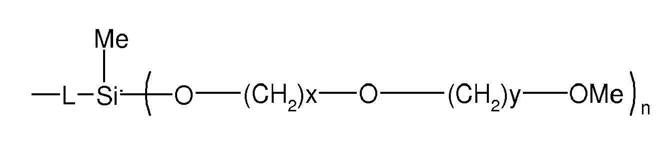 avec n=2

-L-Si-(O-(CH₂)x-O-(CH₂)y-OMe)ₘ

avec m=3
dans lesquelles x = 1 à 10, de préférence 2; et y = 1 à 10, de préférence 2; et L étant tel que défini ci-dessus.
De préférence, dans ces formules, L est un radical alkylène en C1-C8, linéaire ou ramifié, notamment méthylène, éthylène, propylène, butylène et notamment n-butylène, octylène ou de formule -CH₂-CH(CH₃)-.

Dans un mode de réalisation particulier, R et R', identiques ou différents, sont tous les deux de formule (III).

Dans un autre mode de réalisation, l'un des radicaux R ou R' représente un radical alkyle en C₁ à C₃₀, linéaire, ramifié et/ou cyclique, saturé ou insaturé, et comprenant éventuellement 1 à 3 hétéroatomes choisis parmi O, S, F et N.
Ceci s'avère particulièrement avantageux pour conférer un caractère universel aux composés de formule (I), c'est-à-dire leur permettre de texturer à la fois des milieux carbonés polaires ou apolaires, des milieux siliconés linéaires ou cycliques, des huiles mixtes c'est-à-dire carbonées partiellement siliconées, ainsi que leurs mélanges,.
La chaîne carbonée peut être interrompue par le/les hétéroatomes et/ou peut comprendre un substituant comprenant ledit/lesdits hétéroatomes, notamment sous forme de groupement carbonyle (-CO-), d'un ou plusieurs radicaux hydroxy (-OH), et/ou d'un radical ester -COOR" avec R" = radical alkyle, linéaire ou ramifié, ayant 1 à 8 atomes de carbone.

Ainsi, ledit radical R ou R' peut être un groupement choisi parmi:

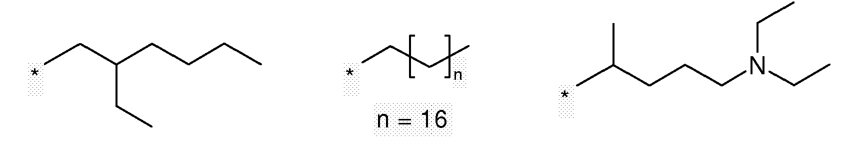

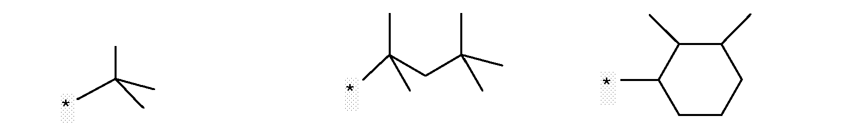

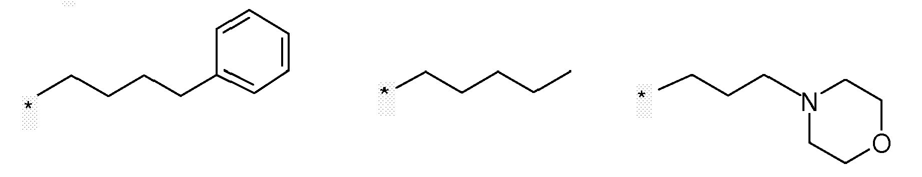

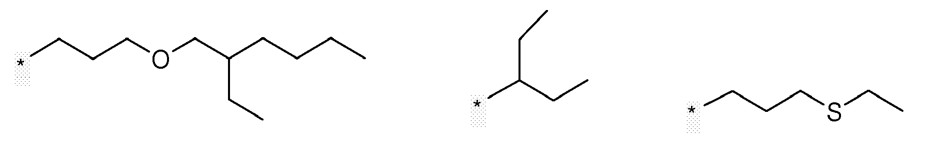

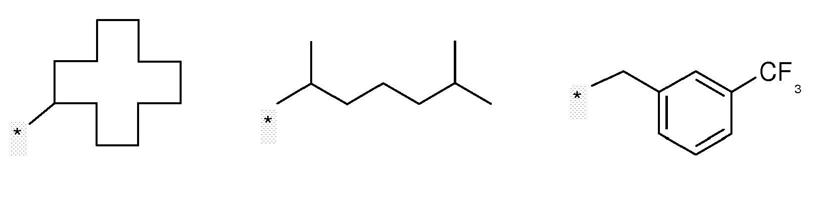

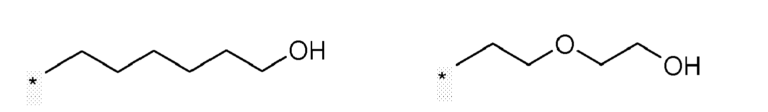

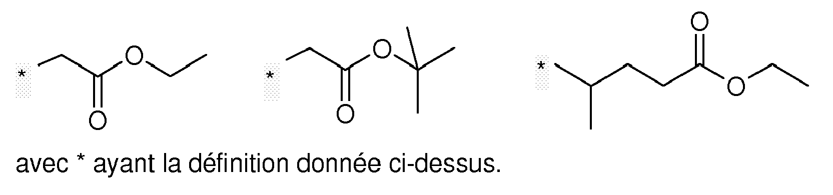

Dans un mode de réalisation tout particulièrement préféré, R ou R' représente un radical alkyle ramifié, notamment monoramifié, de préférence non cyclique, saturé ou insaturé, comprenant 3 à 16 atomes de carbone, notamment 4 à 12, voire 4 à 8 atomes de carbone, et comprenant éventuellement 1 à 3 hétéroatomes choisis parmi O, S, F et/ou N, de préférence O et/ou N.
En particulier, R ou R' peuvent être des radicaux tertio-butyle ou 2-éthylhexyle ou de formule :

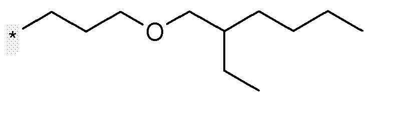

Le fait que l'un des radicaux R ou R' soit ramifié permet notamment d'obtenir une bonne compatibilité des composés avec une plus large gamme de solvants, aussi bien siliconés qu'hydrocarbonés.

Lorsque le composé de formule (I) comprend un radical R qui est un radical alkyle, et donc un radical R' qui est de formule (III), le rapport entre n_{R} et n_{R'} est de préférence compris entre 5/95 et 95/5, par exemple entre 10/90 et 90/10, en particulier entre 40/60 et 85/15, notamment entre 50/50 et 80/20, voire entre 60/40 et 75/25;
avec n_{R} étant le nombre de moles d'amine NH₂-R et n_{R'} étant le nombre de moles d'amine NH₂-R' utilisées pour préparer le composé de formule (I).

Les composés selon l'invention peuvent se présenter sous forme de sels et/ou d'isomères de composés de formule (I).

De préférence, les composés selon l'invention ont une masse moléculaire inférieure à 5000, notamment inférieure à 3000, de préférence comprise entre 300 et 5000, voire entre 400 et 3000; ceci permet de les différentier des polymères siliconés, notamment PDMS, pouvant porter des fonctions urée, et qui peuvent être employés, notamment en mélange, dans des systèmes gélifiants des huiles de silicone. L'avantage lié à l'utilisation de molécules de faible poids moléculaire réside dans leur meilleure compatibilité, et solubilité, vis-à-vis des huiles carbonées et siliconées, notamment polaires, ainsi que leur facilité de formulation et de mise en oeuvre, par rapport aux polymères.

Préférentiellement, les composés de formule (1) selon l'invention peuvent être choisis parmi, seuls ou en mélange, les composés suivants, ainsi que leurs sels et isomères :

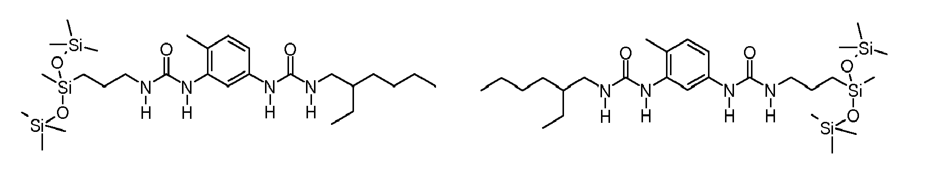

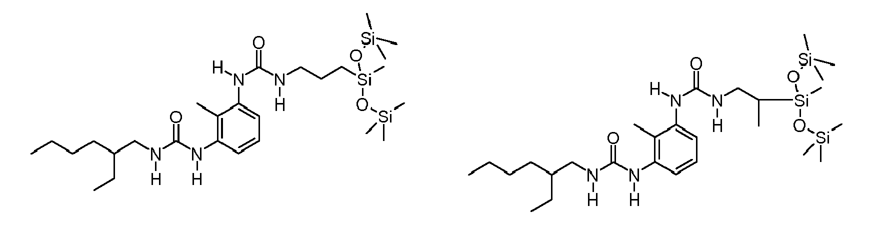

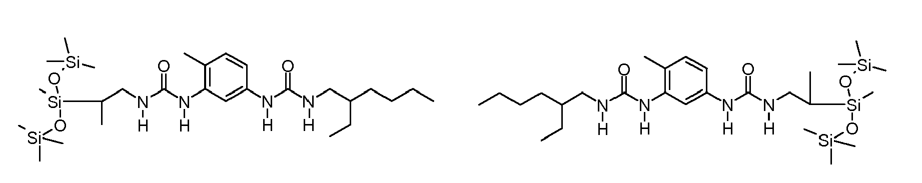

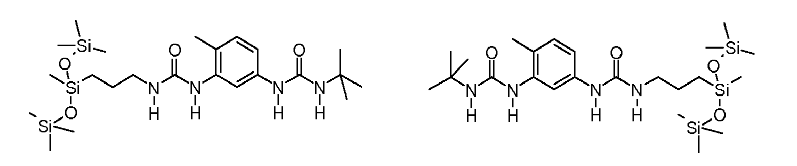

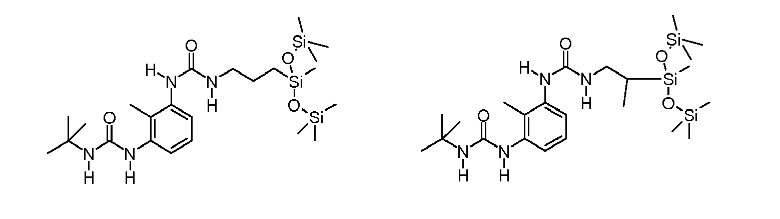

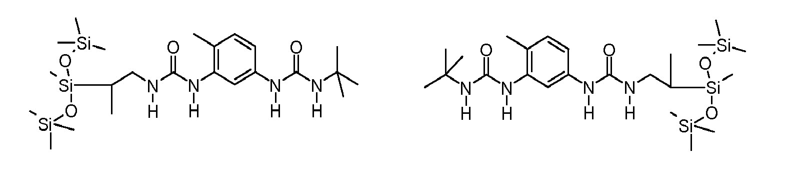

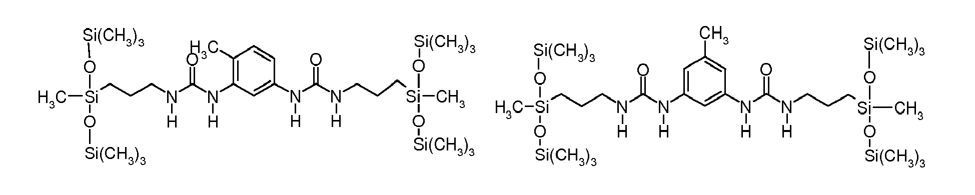

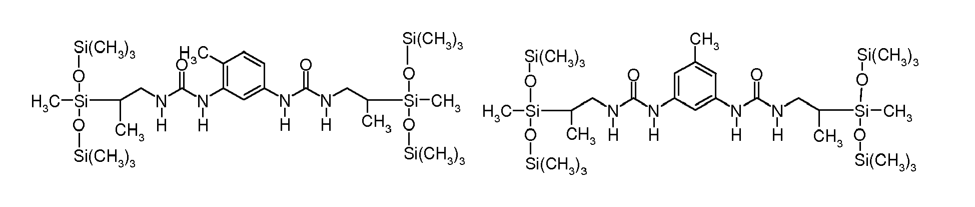

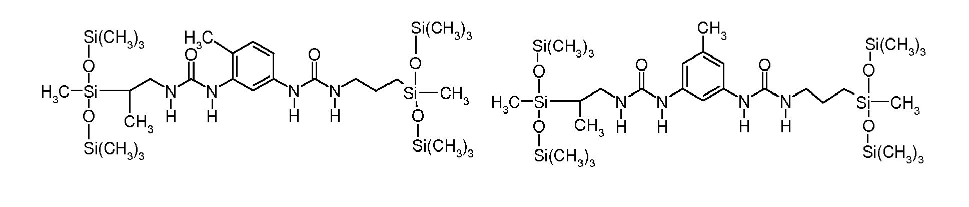

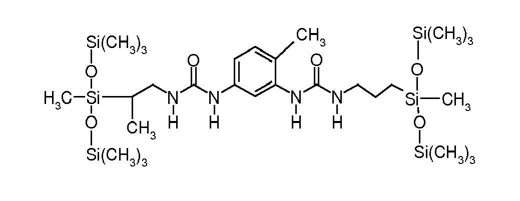

D'une manière générale, les composés de formule générale (I) selon l'invention peuvent être préparés par réaction entre au moins un di-isocyanate de formule OCN-A-NCO, et au moins une amine primaire R-NH₂, avec A et R tels que définis précédemment.
Lorsque les radicaux R et R' sont différents, on fait réagir le diisocyanate avec un mélange d'au moins deux amines primaires : R-NH₂ + R'-NH₂.

Le diisocyanate OCN-A-NCO peut se présenter sous forme de mélange d'isomères de position du substituant R₁ sur le groupement A, notamment dans des proportions 95/5 ou 80/20 (isomère 2,4 TDI)/(isomère 2,6 TDI).

De préférence la/les amines utilisées sont dans un rapport molaire de 2 à 3 équivalents, notamment 2,1 à 2,5, voire 2,2 équivalents pour un équivalent de diisocyanate(s). Le schéma réactionnel général est le suivant :

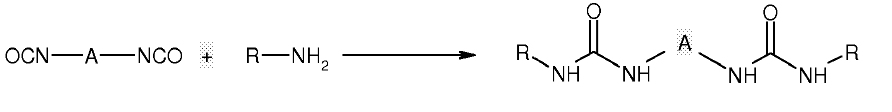

II est bien évidemment possible d'employer un mélange d'amines primaires, et notamment deux amines primaires.
Dans le cas particulier où deux amines primaires sont utilisées pour la réaction, le rapport molaire n_{R}/n_{R'} peut être compris entre 5/95 et 95/5; ce rapport dépend bien évidemment de la nature chimique de chacune des amines, et sera aisément déterminé par l'homme du métier sur la base de ses connaissances.
Ceci est particulièrement vrai lorsque R et R' sont tous les deux de formule (III).
Lorsque R est un radical alkyle, et donc R' de formule (III), ledit rapport molaire n_{R}/n'_{R'} peut être compris entre 5/95 et 95/5, par exemple entre 15/85 et 90/10, en particulier entre 40/60 et 85/15, notamment entre 50/50 et 80/20;
avec n_{R} étant le nombre de moles d'amine NH₂-R et n_{R'} étant le nombre de moles d'amine NH₂-R' utilisées pour préparer le composé de formule (I).
On peut notamment citer le mélange d'amines primaires commercialisé par Clariant sous la dénomination 'aminopropylbis(triméthylsiloxy)silane', qui correspond à un mélange comprenant 80 à 99,5% en poids, notamment 90 à 99% en poids d'une première amine primaire de formule NH₂-(CH₂)₃-Si[OSi(CH₃)₃]₂Me et 0,5 à 20% en poids, notamment 1 à 10% en poids d'une deuxième amine primaire, de formule NH₂-CH₂-CH(CH₃)-Si[OSi(CH₃)₃]₂Me.

La réaction est généralement effectuée sous atmosphère inerte par exemple sous argon, en milieu anhydre avec par exemple une température du milieu réactionnel qui est maintenue entre 15°C et 40°C.
Le diisocyanate peut être mis en solution dans un solvant anhydre tel que la tétrahydrofurane, la 2-méthyl-tétrahydrofurane, la N-méthylpyrrolidone, l'acétate de butyle ou encore la méthyl-éthylcétone à une concentration pouvant aller de 1 à 30% en poids, de préférence de 2 à 20%, voire de 4 à 10% en poids.
Une solution contenant la/les amines peut être préparée dans le même solvant que le diisocyanate, à une concentration pouvant aller de 0,1 à 99,9% en poids. La température du milieu réactionnel ne doit préférentiellement pas dépasser 40°C et la concentration de l'amine ainsi que la vitesse d'addition de la solution contenant l'amine sont préférentiellement ajustées à cette nécessité.
Le milieu réactionnel peut être laissé sous agitation par exemple pendant 30 minutes à 12 heures. Le contrôle de l'avancement de la réaction peut être réalisé par spectrométrie infra-rouge (notamment en observant la disparition de la bande NCO entre 2250 et 2280 cm⁻¹).
En fin de réaction, on peut verser le milieu réactionnel dans une grande quantité d'eau acide (notamment un pH 3-4 par HCl). On obtient alors un précipité, généralement blanc, qui est filtré, lavé par exemple plusieurs fois notamment à l'eau, et séché sous pression réduite, notamment sous vide ou lyophilisé.
Le précipité correspond au composé de formule (1) attendu, ou au mélange de composés de formule (1) attendu, et peut être caractérisé par spectrométrie RMN (¹H et ou ¹³C) et/ou par HPLC couplé à la masse.

Le composé peut être utilisé tel quel pour la texturation du milieu huileux considéré.
En effet, les composés de formule (1), seuls ou en mélange en toutes proportions, peuvent se solubiliser dans une grande diversité d'huiles et s'avèrent donc efficaces pour texturer les huiles ou mélange d'huiles considérées, en lui conférant les propriétés physiques et/ou chimiques souhaitées.
Le composé de formule (I) est avantageusement soluble à une température inférieure ou égale à 50°C, voire inférieure ou égale à 30°C, et notamment à température ambiante (25°C), dans les phases grasses liquides usuellement employées en cosmétique, et donc dans la phase grasse à texturer.

Le composé selon l'invention trouve donc une application toute particulière dans les compositions cosmétiques ou pharmaceutiques usuelles, notamment en tant qu'agent texturant, épaississant, voire gélifiant, de la phase grasse liquide comprise dans ladite composition.

Ledit composé de formule (1), seul ou en mélange, peut être présent dans lesdites compositions en une quantité efficace, c'est-à-dire en une quantité nécessaire et suffisante pour obtenir la texturation de la phase grasse liquide considérée dans la composition selon l'invention.

Par "phase grasse liquide texturée", on entend que ladite phase grasse prend l'état d'un gel ou d'un liquide épaissi. Elle peut s'écouler sous son propre poids. Elle peut se déformer à volume constant si on exerce une contrainte.
Cette texturation se traduit en particulier par une augmentation de la viscosité due notamment à l'introduction d'au moins un composé de formule (1).

Ainsi, les compositions selon l'invention peuvent comprendre de 0,01 à 20% en poids, notamment de 0,05 à 15% en poids, voire de 0,1 à 10% en poids, mieux de 1 à 8% en poids, et encore mieux de 2 à 5% en poids, de composé(s) de formule (I) par rapport au poids total de la composition.
La quantité efficace de composé(s) de formule (1) peut notamment représenter de 0,01 à 20% en poids, notamment de 0,05 à 15% en poids, voire de 0,1 à 10% en poids, mieux de 1 à 8% en poids, et encore mieux de 2 à 5% en poids, par rapport au poids de ladite phase grasse liquide.
Il est clair que cette quantité efficace est susceptible de varier significativement selon, entre autre, la nature du composé dérivé bis-urée, le fait qu'il soit utilisé à l'état pur ou en mélange avec d'autres dérivés bis-urée de formule (1), et la nature de la phase grasse liquide.

Les compositions cosmétiques ou pharmaceutiques selon l'invention comprennent en outre, un milieu physiologiquement acceptable, notamment cosmétiquement acceptable, c'est-à-dire compatible avec les matières kératiniques telles que la peau du visage ou du corps, les lèvres, les cheveux, les cils, les sourcils, le cuir chevelu et les ongles.

La composition selon l'invention comprend, dans un milieu physiologiquement acceptable, au moins une phase grasse liquide, susceptible d'être texturée.
Par phase grasse liquide, on entend au sens de l'invention une phase grasse liquide à température ambiante (25°C) et pression atmosphérique (760 mm de Hg), composée d'un ou plusieurs composé lipophiles choisis parmi les huiles siliconées, les huiles carbonées et les solvants carbonés, tels que définis ci-après, liquides à température ambiante. La phase grasse liquide peut représenter 0,1 à 90% en poids de la composition, notamment de 0,5 à 35% en poids, en particulier de 1 à 30% en poids, voire 2 à 20% en poids, encore mieux 3 à 15% en poids, du poids total de la composition.

La phase grasse liquide peut donc comprendre une huile et/ou un solvant qui peut être carboné, notamment hydrocarboné, et/ou fluoré; cette huile ou solvant peut notamment être choisi parmi :
1/ les esters des acides monocarboxyliques avec les monoalcools et polyalcools; avantageusement, ledit ester répond à la formule suivante : R'₁-CO-O-R'₂ où :
   R'₁ représente un radical alkyle linéaire ou ramifié de 1 à 40 atomes de carbone, de préférence de 7 à 19 atomes de carbone, comprenant éventuellement une ou plusieurs double liaisons éthyléniques, éventuellement substitué et dont la chaîne hydrocarbonée peut être interrompue par un ou plusieurs hétéroatomes choisis parmi N et O et/ou une ou plusieurs fonctions carbonyles, et
   R'₂ représente un radical alkyle linéaire ou ramifié de 1 à 40 atomes de carbone, de préférence de 3 à 30 atomes de carbone et mieux de 3 à 20 atomes de carbone, comprenant éventuellement une ou plusieurs double liaisons éthyléniques, éventuellement substitué et dont la chaîne hydrocarbonée peut être interrompue par un ou plusieurs hétéroatomes choisis parmi N et O et/ou une ou plusieurs fonctions carbonyles.
   Par "éventuellement substitué", on entend que R'₁ et/ou R'₂ peuvent porter un ou plusieurs substituants choisis, par exemple, parmi les groupements comprenant un ou plusieurs hétéroatomes choisi parmi O et/ou N, tels que amino, amine, alcoxy, hydroxyle.
   Des exemples des groupes R'₁ sont ceux dérivés des acides gras de préférence supérieur choisis dans le groupe constitué des acides acétique, propionique, butyrique, caproïque, caprylique, pélargonique, caprique, undécanoïque, laurique, myristique, palmitique, stéarique, isostéarique, arachidique, béhénique, oléique, linolénique, linoléïque, oléostéarique, arachidonique, érucique, et de leurs mélanges.
   De préférence, R'₁ est un groupe alkyle ramifié non substitué de 4 à 14 atomes de carbone, de préférence de 8 à 10 atomes de carbone et R₂ est un groupe alkyle ramifié non substitué de 5 à 15 atomes de carbone, de préférence de 9 à 11 atomes de carbone.
   On peut en particulier citer, de préférence, les esters en C₈-C₄₈, éventuellement incorporant dans leur chaîne hydrocarbonée un ou plusieurs hétéroatomes parmi N et O et/ou une ou plusieurs fonctions carbonyle; et plus particulièrement l'huile de purcellin (octanoate de cétostéaryle), l'isononanoate d'isononyle, le myristate d'isopropyle, le palmitate d'éthyl-2-hexyle, le stéarate d'octyl-2-dodécyle, l'érucate d'octyl-2-dodécyle, le l'isostéarate d'isostéaryle, et les heptanoates, octanoates, décanoates ou ricinoléates d'alcools ou de polyalcools, par exemple d'alcools gras, ainsi que le N-lauroyl sarcosinate d'isopropyle (notamment Eldew-205SL d'Ajinomoto).
2/ les huiles fluorées telles que les perfluoropolyéthers, les perfluoroalcanes comme la perfluorodécaline, les perfluorodamantanes, les monoesters, diesters et triesters de perfluoroalkylphosphates et les huiles esters fluorés.
3/ les huiles végétales hydrocarbonées à forte teneur en triglycérides constitués d'esters d'acides gras et de glycérol dont les acides gras peuvent avoir des longueurs de chaînes variées, ces dernières pouvant être linéaires ou ramifiées, saturées ou insaturées ; ces huiles sont notamment les huiles de germe de blé, de maïs, de tournesol, de karité, de ricin, d'amandes douces, de macadamia, d'abricot, de soja, de colza, de coton, de luzerne, de pavot, de potimarron, de sésame, de courge, d'avocat, de noisette, de pépins de raisin ou de cassis, d'onagre, de millet, d'orge, de quinoa, d'olive, de seigle, de carthame, de bancoulier, de passiflore, de rosier muscat ; ou encore les triglycérides des acides caprylique/caprique comme ceux vendus par la société Stearinerie Dubois ou ceux vendus sous les dénominations Miglyol 810, 812 et 818 par la société Dynamit Nobel,
4/ les éthers en C₆ à C₄₀,
5/ les acides gras en C₈-C₃₂, comme l'acide oléique, linoléique ou linolénique,
6/ les alcools dits 'gras', et notamment les monoalcools, en C6-C32 comme l'alcool oléïque et l'octyldodécanol;
7/ les hydrocarbures ou fluorocarbures, linéaires ou ramifiés, d'origine synthétique ou minérale, comme les huiles de paraffine (telles que les isoparaffines en C₈-C₁₆, l'isododécane, l'isohexadécane) et leurs dérivés, la vaseline, les polydécènes, les polyisobutènes hydrogénés tel que le squalane, et leurs mélanges.
   On peut en particulier citer les alcanes, linéaires ou ramifiés, en C₆-C₄₈, notamment volatiles.
8/ les huiles bifonctionnelles, comprenant deux fonctions choisies parmi ester et/ou amide et comprenant de 6 à 30 atomes de carbone, notamment 8 à 28 atomes de carbone, mieux de 10 à 24 carbones, et 4 hétéroatomes choisis parmi O et N; de préférence les fonctions amide et ester étant dans la chaîne;
9/ et leurs mélanges.

La phase grasse liquide peut également comprendre une ou plusieurs huiles siliconées, qui peuvent être volatiles ou non volatiles. Au sens de l'invention, les huiles volatiles présentent à température ambiante (25°C) et pression atmosphérique (760 mm de Hg) une pression de vapeur allant de 0,02 mm à 300 mm de Hg (2,66 Pa à 40 000 Pa) et mieux allant de 0,1 à 90 mm de Hg (13 Pa à 12 000 Pa). Les huiles non volatiles correspondent alors à une pression de vapeur inférieure à 0,02 mm de Hg (2,66 Pa).

La ou les huiles siliconées peuvent être choisies parmi :
- les huiles siliconées volatiles linéaires ou cycliques, telles que les polydiméthylsiloxanes (PDMS) linéaires ou cycliques ayant de 3 à 7 atomes de silicium.
   A titre d'exemple de telles huiles volatiles, on peut citer les composés tels que l'octyltriméthicone, l'hexyltriméthicone, la décaméthylcyclopentasiloxane (ou D5), l'octaméthylcyclotétrasiloxane (ou D4), la dodécaméthylcyclohexasiloxane (ou D6), la décaméthyltétrasiloxane (ou L4) notamment la KF 96 A de Shin Etsu, les PDMS (polydiméthylsiloxane) volatiles et notamment la DC 200 (1,5 cSt) de Dow Corning, la PDMS DC 200 (2 cSt) de Dow Corning, la PDMS DC 200 (5 cSt) de Dow Corning, la PDMS DC 200 (3 cSt) de Dow Corning, et/ou leurs mélanges.
   On peut également citer l'heptaméthyloctyltrisiloxane, le dodécaméthylpentasiloxane, le polyméthylcétyldiméthylsiloxane et/ou leurs mélanges.
   L'huile siliconée volatile peut aussi être choisie dans le groupe des huiles siliconées fluorées telles que les silicones à groupes alkyle et perfluoralkyle, les silicones à groupes latéraux oxyéthylénés/oxypropylénés (OE/PP) et à groupes perfluorés, les silicones à groupes latéraux perfluorés ou polyfluorés et à groupes latéraux glycérolés, et les perfluoroalkylméthylphénylsiloxanes.
- les huiles siliconées non volatiles tels que les polydiméthylsiloxanes (PDMS), des polyalkylméthylsiloxanes, des diméthicone copolyols, des alkylméthicone copolyols, la cétyldiméthicone, des silicones à groupes alkylglycéryl éthers, des silicones à groupes amines latéraux et le dilauroyltriméthylol propane siloxysilicate. Les groupements alkyle de ces huiles ont notamment de 2 à 24 atomes de carbone.
   Les huiles siliconées non volatiles utilisables peuvent être en particulier les polydiméthylsiloxanes (PDMS) non volatils, linéaires, liquides à température ambiante; les polydiméthylsiloxanes comportant des groupements alkyle, alcoxy ou phényle, pendants et/ou en bout de chaîne siliconée, groupements ayant chacun de 2 à 24 atomes de carbone; les silicones phénylées comme les phényl triméthicones, les phényl diméthicones, les phényl triméthylsiloxy diphénylsiloxanes, les diphényl diméthicones, les diphényl méthyldiphényl trisiloxanes, les 2-phényléthyl triméthylsiloxysilicates, la cétyldiméthicone, les silicones à groupes alkylglycéryl éthers, les silicones à groupe amines latéraux, les silicones fluorées à groupement(s) pendant(s) ou en bout de chaîne ayant de 1 à 12 atomes de carbone dont tout ou partie des atomes d'hydrogène est substitué par des atomes de fluor, les diméthiconols et/ou leurs mélanges.
- et leurs mélanges.

La phases grasse liquide à texturer peut comprendre un mélange d'huiles carbonée et siliconée, qui peut donc avantageusement être texturé par les composés de formule (I) selon l'invention.

De préférence, les compositions selon l'invention comprennent une phase grasse liquide qui comprend au moins un composé lipophile choisi parmi :
- les monoalcools en C₆-C₃₂, de préférence en C₈-C₂₈, mieux en C₁₂-C₂₆ et préférentiellement l'octyldodécanol;
- les alcanes ramifiés en C₆-C₃₂, de préférence en C₈-C₂₈, mieux en C₁₂-C₂₆ et préférentiellement l'isododécane ou le parléam de formule -(CH₂-CH(CH₃))ₙ-avec n étant un entier variant de 4 à 8;
- les alcanes linéaires en C₁₃-C₄₈, de préférence en C₁₈-C₄₀, mieux en C₂₀-C₃₂;
- les huiles bifonctionnelles, comprenant deux fonctions choisies parmi ester et/ou amide et comprenant de 6 à 30 atomes de carbone, notamment 8 à 28 atomes de carbone, mieux de 10 à 24 carbones, et 4 hétéroatomes choisis parmi O et N; de préférence les fonctions amide et ester étant dans la chaîne; en particulier l'huile bifonctionnelle peut être le N-lauroylsarcosinate d'isopropyle de formule suivante : 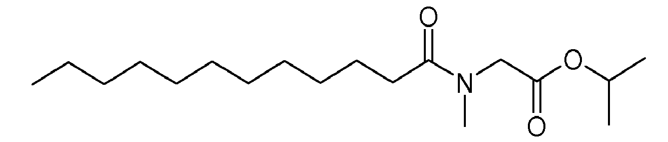 et plus particulièrement celui commercialisé sous la dénomination Eldew SL-205^{®} d'Ajinomoto.
- les esters en C₈-C₄₈, éventuellement incorporant dans leur chaîne hydrocarbonée un ou plusieurs hétéroatomes parmi N et O et/ou une ou plusieurs fonctions carbonyle; et plus particulièrement l'huile de purcellin (octanoate de cétostéaryle), l'isononanoate d'isononyle, le myristate d'isopropyle, le palmitate d'éthyl-2-hexyle, le stéarate d'octyl-2-dodécyle, l'érucate d'octyl-2-dodécyle, le l'isostéarate d'isostéaryle, et les heptanoates, octanoates, décanoates ou ricinoléates d'alcools ou de polyalcools, par exemple d'alcools gras;
- les huiles siliconées, volatiles ou non, et notamment les polydiméthylsiloxanes (PDMS) linéaires ou cycliques ayant de 3 à 7 atomes de silicium tels que la décaméthylcyclopentasiloxane, l'octaméthylcyclotétrasiloxane, la dodécaméthylcyclohexasiloxane et la décaméthyltétrasiloxane; et les silicones phénylées comme les phényl triméthicones;
- leurs mélanges.

Ces composés lipophiles préférés peuvent représenter 20 à 100%, voire de 40 à 99 %, notamment de 60 à 95% en poids de la phase grasse liquide totale.

En effet, les inventeurs ont constaté que les associations d'au moins un composé de type bis-urée de formule (I) avec au moins un composé lipophile tel que défini ci-dessus, donnent des résultats particulièrement satisfaisants au sens de l'invention.

Les compositions selon l'invention peuvent en outre comprendre au moins un corps gras solide, qui peut être choisi parmi les cires et/ou les composés pâteux. Plus particulièrement, les compositions selon l'invention peuvent comprendre de 0,1% à 40% en poids, notamment de 0,1% à 30% en poids, et plus particulièrement de 0,5% à 25% en poids de corps gras solides par rapport au poids total de la composition.
Parmi les corps gras pâteux, on peut citer les gommes de silicone.
Par cire au sens de la présente invention, on entend un composé lipophile, solide à température ambiante (25°C), à changement d'état solide/liquide réversible, ayant un point de fusion supérieur ou égal à 30°C, mieux supérieur à 45°C, et pouvant aller jusqu'à 120°C. Les cires peuvent être d'origine végétale, animale, minérale ou de synthèse; elles peuvent être carbonées, notamment hydrocarbonées, fluorées et/ou siliconées. On peut notamment citer la cire d'abeilles, la cire de Carnauba, la cire de Candellila, la paraffine, les cires microcristallines, la cérésine, l'ozokérite; les cires synthétiques comme les cires de polyéthylène ou de Fischer Tropsch, les cires de silicones comme les alkyl ou alkoxy-diméticone ayant de 16 à 45 atomes de carbone; ainsi que leurs mélanges.

La composition peut comprendre une phase aqueuse qui peut être constituée essentiellement d'eau ou qui peut comprendre un mélange d'eau et de solvant organique miscible à l'eau, et notamment choisis parmi les monoalcools ayant de 1 à 5 atomes de carbone tels que l'éthanol ou l'isopropanol; les glycols ayant de 2 à 8 atomes de carbone tels que le propylène glycol, l'éthylène glycol, le 1,3-butylène glycol, le dipropylène glycol, les cétones en C₃-C₄, et les aldéhydes en C₂-C₄.
La phase aqueuse peut être présente à une teneur de 1 % à 95% en poids, notamment de 3% à 80% en poids, et en particulier de 5% à 60% en poids, par rapport au poids total de la composition.
La composition selon l'invention peut également être exempte d'eau (0%).

La composition selon l'invention peut comprendre au moins une matière colorante, organique ou inorganique, et qui peut être choisie parmi les colorants lipophiles, les colorants hydrophiles, les pigments, les nacres, les matériaux à effet optique spécifique, et leurs mélanges. Cette matière colorante peut être présente à raison de 0,01 à 50% en poids par rapport au poids total de la composition, en particulier de 0,5 à 40% et plus particulièrement de 1 à 25%, et notamment de 5 à 20% en poids par rapport au poids total de la composition.
La composition selon l'invention peut comprendre en outre en outre une ou plusieurs charges, notamment en une teneur allant de 0,01% à 50% en poids, par rapport au poids total de la composition, de préférence allant de 0,05% à 30% en poids.

La composition selon l'invention peut également comprendre des ingrédients couramment utilisés en cosmétique, tels que les vitamines, les épaississants, les gélifiants, les oligo-éléments, les adoucissants, les séquestrants, les parfums, les agents alcalinisants ou acidifiants, les conservateurs, les filtres solaires, les tensioactifs, les anti-oxydants, les agents anti-chutes des cheveux, les agents anti-pelliculaires, les agents propulseurs, les céramides, les polymères, les actifs cosmétiques, ou leurs mélanges. Bien entendu, l'homme du métier veillera à choisir ce ou ces éventuels composés complémentaires, et/ou leur quantité, de manière telle que les propriétés avantageuses de la composition selon l'invention ne soient pas, ou substantiellement pas, altérées par l'adjonction envisagée.

La composition selon l'invention peut se présenter notamment sous forme de suspension, de dispersion, de solution notamment organique, de gel, d'émulsion, notamment émulsion huile-dans-eau (H/E) ou eau-dans-huile (E/H), ou multiple (E/H/E ou polyol/H/E ou H/E/H), sous forme de crème, de pâte, de mousse, de dispersion de vésicules notamment de lipides ioniques ou non, de lotion biphase ou multiphase, de spray, de poudre, de stick ou bâton.
L'homme du métier pourra choisir la forme galénique appropriée, ainsi que sa méthode de préparation, sur la base de ses connaissances générales, en tenant compte d'une part de la nature des constituants utilisés, notamment de leur solubilité dans le support, et d'autre part de l'application envisagée pour la composition.

La composition selon l'invention peut être une composition de maquillage, notamment un produit pour le teint tel qu'un fond de teint, un fard à joues ou à paupières; un produit pour les lèvres tel qu'un rouge à lèvres ou un soin des lèvres; un produit anti-cernes; un blush, un mascara, un eye-liner; un produit de maquillage des sourcils, un crayon à lèvres ou à yeux; un produit pour les ongles tel qu'un vernis à ongles ou un soin des ongles; un produit de maquillage du corps ou des cheveux (mascara ou laque pour cheveux).
La composition selon l'invention peut être une composition de protection ou de soin de la peau du visage, du cou, des mains ou du corps, notamment une composition anti-rides, anti-fatigue, anti-âge, permettant de donner un coup d'éclat à la peau, une composition hydratante ou traitante; une composition anti-solaire ou après-soleil ou de bronzage artificiel.
La composition selon l'invention peut être également un produit capillaire, notamment pour la coloration, le soin, l'hygiène des cheveux, le coiffage, le maintien de la coiffure ou la mise en forme des cheveux. Les compositions capillaires sont de préférence des shampooings, des gels, des lotions de mise en plis, des lotions pour le brushing, des compositions de soin ou d'hygiène capillaire ou des compositions de fixation et de coiffage telles que les laques, les gels ou les sprays.

La composition selon l'invention trouve une application toute particulière dans le domaine des compositions de maquillage, et notamment dans les fonds de teint, les mascaras et les rouges à lèvres.

La composition selon l'invention peut être fabriquée par les procédés connus, généralement utilisés dans le domaine cosmétique ou pharmaceutique.

L'invention a aussi pour objet un procédé de traitement cosmétique, notamment de maquillage, de nettoyage, de protection solaire, de mise en forme, de coloration et/ou de soin, des matières kératiniques, notamment de la peau du corps ou du visage, des lèvres, des ongles, des sourcils, des cheveux et/ou des cils, comprenant l'application sur lesdites matières d'une composition cosmétique telle que définie précédemment.

L'invention est illustrée plus en détail dans les exemples qui suivent, donnés à titre d'illustration.

### Procédé général de préparation

On a effectué différentes préparations de composés de type bis-urées, comportant au moins un motif siliconé et au moins un motif non siliconé.
Les différents mélanges sont obtenus en une étape, le schéma général de synthèse étant représenté ci-dessous :

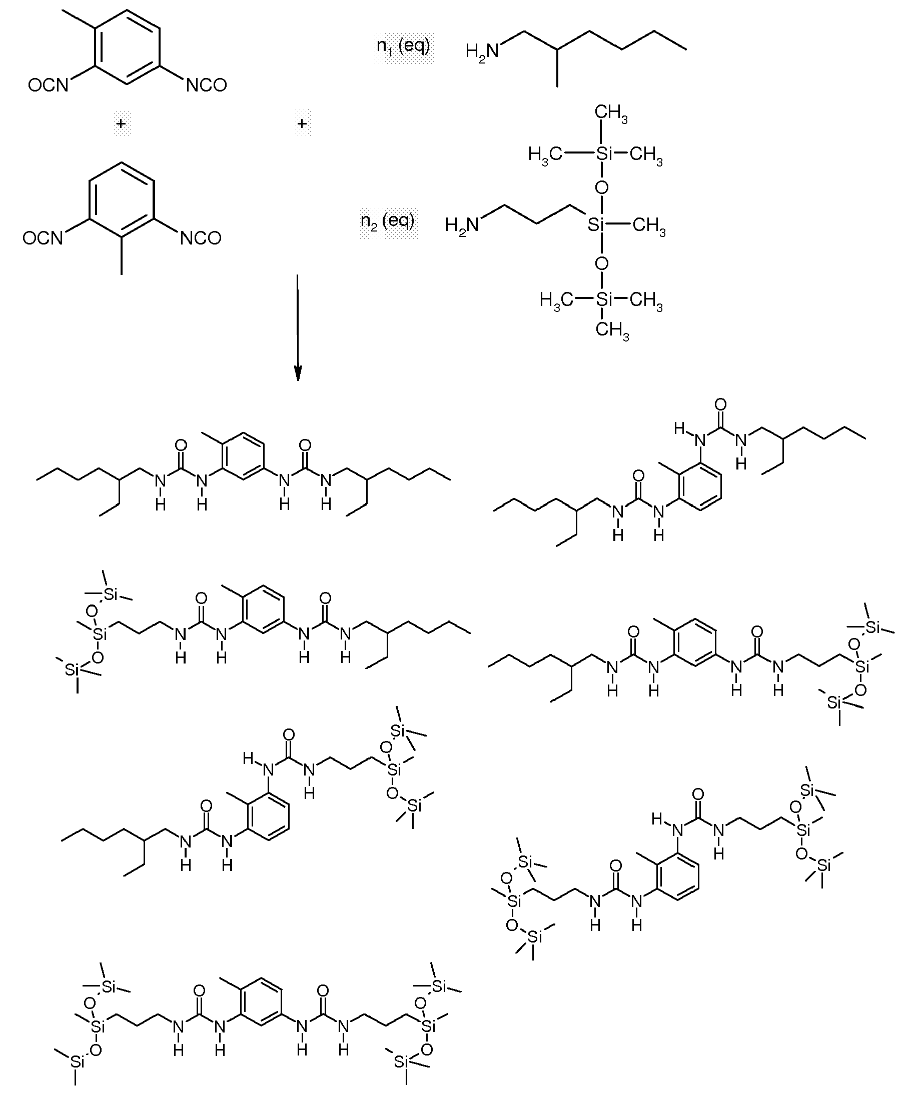

Différents mélanges en fonction du rapport n₁ (nombre de mole de 2-éthyl hexylamine) sur n₂ (nombre de mole de 3-aminopropylméthylbis(triméthylsiloxy) silane) et du pourcentage d'isomère du toluène diisocyanate (TDI) (95/5 ou 80/20) sont préparés.

Ces mélanges ont été préparés selon un même mode opératoire tel que décrit ci-après. Les quantités spécifiques retenues pour chaque matière première de départ sont précisées dans les exemples.

On mélange le diisocyanate de toluilène en solution dans le THF anhydre avec 2,2 équivalents d'amine en solution dans le THF anhydre.
La réaction est effectuée sous atmosphère inerte (argon) en milieu anhydre avec une température du milieu réactionnel qui est maintenue entre 15°C et 40 °C.
Parallèlement, on prépare une solution d'amine (Y) dans le THF. La température du milieu réactionnel ne devant pas, de préférence, dépasser 40°C, la concentration de l'amine et la vitesse d'addition de la solution d'amine (Y) sont ajustées à cette nécessité. On laisse le milieu réactionnel sous agitation en contrôlant l'avancement de la réaction par spectrométrie infra-rouge (disparition de la bande NCO entre 2250 et 2280 cm⁻¹).
Une fois que le diisocyanate a complètement réagi, le mélange réactionnel est additionné à de l'eau acidifiée (pH 3) avec de l'acide chlorhydrique, le précipité obtenu est filtré, lavé plusieurs fois à l'eau et enfin séché sous vide ou lyophilisé. Une poudre blanche est obtenue et utilisée telle quelle après analyse (HPLC couplée à la masse).

### Exemples 1 et 2 (R = R' = 3-aminopropylméthvlbis(triméthvlsiloxv)silane)

Dans ces exemples, le mélange obtenu au final est caractérisé par HPLC couplée à la masse et par RMN ¹H.

### Exemple 1

Matières premières de départ :
- TDI : 95/5 en (isomère 2,4)/(isomère 2,6); m=5 g (28,71 mmoles)
- n₁=0
- n₂= 63,16 mmoles (2,2 éq.) soit m=17,6 g de 3-aminopropylméthylbis-(triméthylsiloxy)silane, avec un taux d'isomère bêta inférieur à 1%.

### Exemple 2

Matières premières de départ :
- TDI : 80/20 en (isomère 2,4)/(isomère 2,6)
- n₁=0
- n₂= 63,16 mmoles (2,2 éq.) soit m=17,6 g de 3-aminopropylméthylbis-(triméthylsiloxy)silane, avec un taux d'isomère bêta inférieur à 1%.

Le mélange obtenu au final dans les exemples 1 et 2 comprend les bis-urées suivantes:

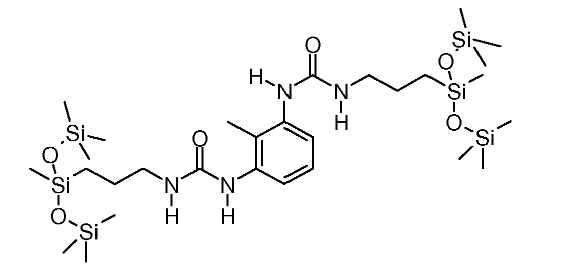

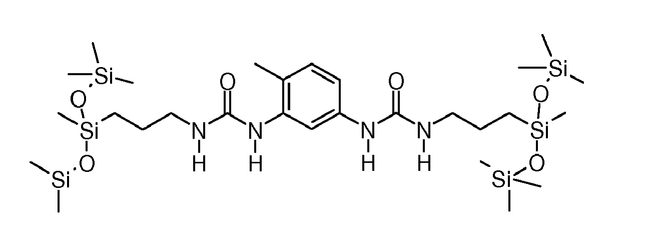

### Exemples 3 et 4 (R = 2-éthylhexyl et R' = 3-aminopropylméthylbis-(triméthylsiloxy)silane)

Dans ces exemples, le mélange obtenu au final est caractérisé par HPLC couplée à la masse et par RMN ¹H.

### Exemple 3

Matières premières de départ :
- TDI : 95/5 en (isomère 2,4)/(isomère 2,6); m=0,317 g (1,82 mmoles)
- n₁= 2 mmoles (1,1 éq.) soit m=0,259 g de 2-éthylhexylamine;
- n₂= 2 mmoles (1,1 éq.) soit m=0,560 g de 3-aminopropylméthylbis-(triméthylsiloxy)silane, avec un taux d'isomère bêta inférieur à 1 %.
   On a donc n_{R}/n'_{R} = 1.

### Exemple 4

Matières premières de départ :
- TDI : 95/5 en (isomère 2,4)/(isomère 2,6); m=50 g (0,29 moles)
- n₁= 0,44 moles (1,5 éq.) soit m=56,87 g de 2-éthylhexylamine;
- n₂= 0,19 moles (0,66 éq.) soit m= 53,13 g de 3-aminopropylméthylbis-(triméthylsiloxy)silane, avec un taux d'isomère bêta inférieur à 1%.
   On a donc n_{R}/n'_{R}= 2,23 (69/31).

Le mélange obtenu au final dans les exemples 3 et 4 comprend les bis-urées suivantes:

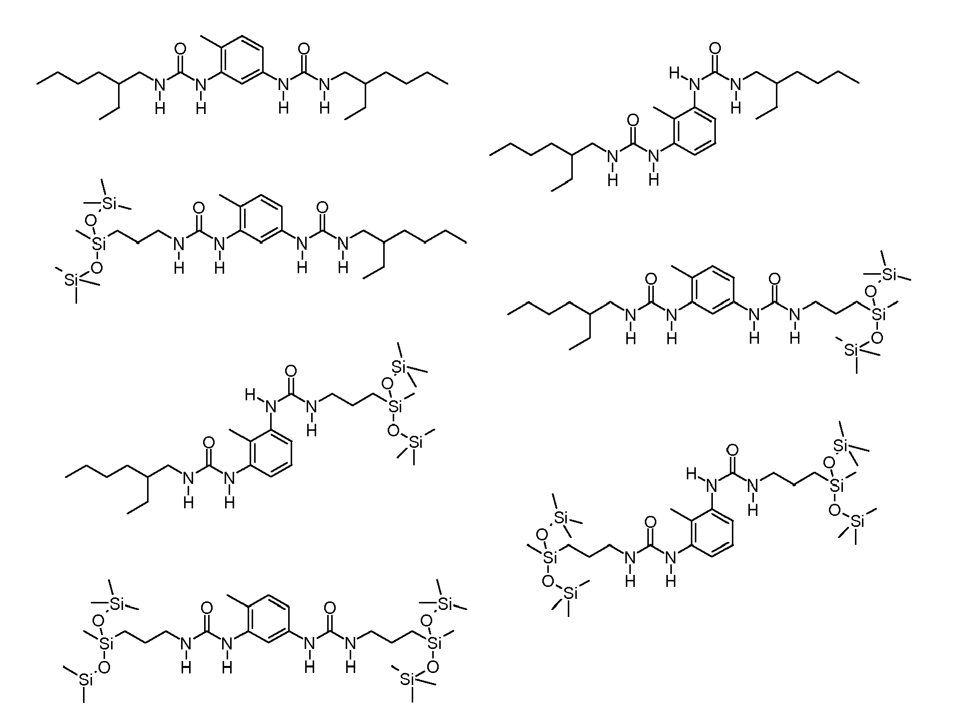

La masse moléculaire des composés comprenant un motif siliconé d'un seul coté est 582, et celle des composés ayant un motif siliconé de chaque côté est 732.

### Exemple 5

Les mélanges préparés aux exemples 1 à 4 ci-dessus, et des composés comparatifs A et B, sont testés à raison de 1 % en poids dans 100 ml de différents composés lipophiles, pour leurs propriétés épaississante ou texturante, à température ambiante (25°C, 1 atm.).
Les composés lipophiles choisis sont : l'isododécane, le parléam, l'octyldodécanol, l'isonanoate d'isononyle, la phényl triméthicone, la silicone volatile D5 et la silicone volatile L4.

Les propriétés épaississantes/texturantes sont jugées satisfaisantes si le mélange de composés se solubilise à température ambiante dans ledit composé lipophile, et si l'on observe une augmentation de la viscosité dudit composé lipophile. Avantageusement, la solution obtenue est généralement limpide, transparente et homogène.

Les mélanges comparatifs A et B sont les suivants :
- Comparatif A: :
   matières premières de départ :
      - TDI : 95/5 en (isomère 2,4)/(isomère 2,6)
      - n₁=2,2 éq. (2-éthylhexylamine) et n₂= 0
- Comparatif B :
   matières premières de départ :
      - TDI : 95/5 en (isomère 2,4)/(isomère 2,6)
      - n₁=1,6 éq. de 2-éthylhexylamine
      - n₂= 0
      - n3 = 0,4 éq. de terbutylamine

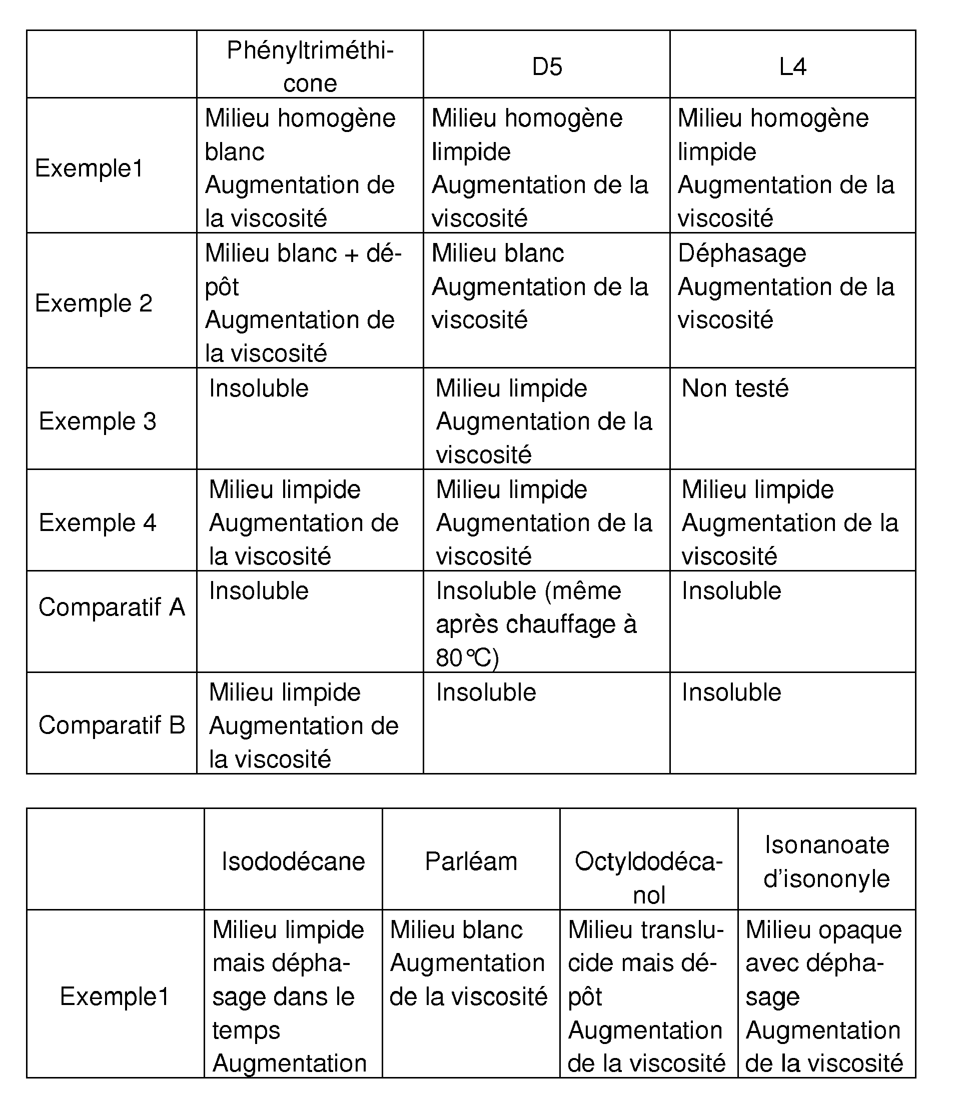

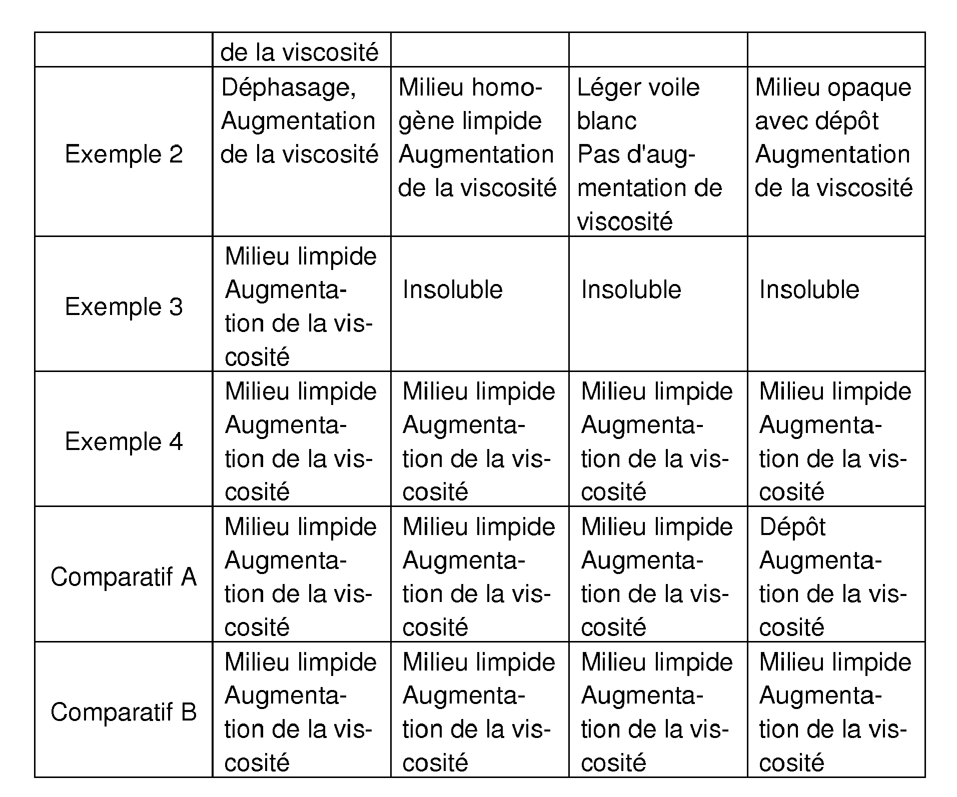

On constate que les composés des exemples 1 à 4, conformes à l'invention, permettent de gélifier une large gamme de solvants siliconés (partiellement siliconé comme la phényltriméthicone, purement siliconé et cyclique comme la D5 et purement siliconé et linéaire comme la L4) et de donner des solutions limpides et homogènes dans la plupart des cas.

Par ailleurs, les composés des exemples 3 et 4, dans lesquels un radical R est carboné (alkyle), et plus particulièrement le composé de l'exemple 4, permettent à la fois de texturer/épaissir une large gamme de solvants siliconés (linéaire et cyclique), mais également des huiles et solvants carbonés, polaires ou apolaires; tout en donnant des solutions limpides et homogènes dans la plupart des cas.
Ceci n'est toutefois pas le cas avec les composés des exemples 1 et 2, purement siliconés; ces composés permettent de texturer/épaissir les huiles carbonées envisagées, mais les solutions obtenues ne sont pas toujours limpides (problème de dépôt ou de déphasage).

### Exemple 6

De manière identique à l'exemple 1, on prépare le composé suivant:
Matières premières de départ :
   - TDI : 95/5 en (isomère 2,4)/(isomère 2,6) ; m= 10g (57,41 mmoles)
   - n₁=0
   - n₂= 126,3 mmoles (2,2 éq.) soit m=35,23 g de 3-aminopropylméthylbis-(triméthylsiloxy)silane, avec un taux d'isomère bêta inférieur à 10% (dosé à 7%); c'est-à-dire un mélange d'amine de formule NH₂-CH₂-CH₂-CH₂-Si[OSi(CH₃)₃]₂Me et d'amine de formule : NH₂-CH₂-CH(CH₃)-Si[OSi(CH₃)₃]₂Me (appelée isomère bêta), la seconde étant présente en une quantité inférieure à 10% en poids.

Le mélange obtenu au final se comporte comme celui de l'exemple 1, qui comprenait au maximum 1% en poids d'isomère bêta : il se solubilise et augmente la viscosité d'huiles siliconées, et en particulier de la D5 et de la L4.

### Exemple 7

De manière identique à l'exemple 1, on prépare le composé suivant :
- TDI : 95/5 en (isomère 2,4)/(isomère 2,6)
- n₁=0;
- n₂= 2,2 éq. de bis-trimethylsilanylmethylamine (amine primaire comportant du silicium).

Le mélange obtenu est insoluble dans tous les solvants mentionnés à l'exemple 5. Ceci montre qu'il ne suffit pas de rajouter un motif silicium dans la structure du composé organogélateur pour obtenir un composé susceptible de gélifier des solvants siliconés et/ou carbonés, et encore moins à la fois des solvants carbonés et siliconés.

### Exemple 8: gel de coiffage

On prépare une composition de gel de coiffage comprenant (% en poids):
- composé de l'exemple 4 3%
- isoeicosane 5%
- trimellitate de tridécyle 5%
- isododécane qsp 100%
On obtient un gel cristal, très épais, qui est un produit de soin capillaire aux qualités tout à fait satisfaisantes.

### Exemple 9 : Fond de teint

On prépare un fond de teint comprenant (% en poids):
- Composé de l'exemple 1 1,5%
- Cyclopentasiloxane 65%
- Dioxyde de titane 7%
- Glycérine 3%
- Nylon-12 2,5%
- Oxydes de fer 2,5%
- bis-PEG/PPG-14/14 dimethicone 1,8%
- Sulfate de magnésium 0,7%
- Isostearyl diglyceryl succinate 0,6%
- Conservateurs qs
- Eau qsp 100%

## Revendications

1. Composition cosmétique ou pharmaceutique comprenant, dans un milieu physiologiquement acceptable, une phase grasse et au moins un composé de formule générale (1), ou l'un de ses sels et/ou isomères : 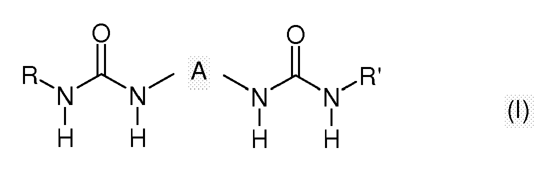 dans laquelle :
- A est un groupement de formule (II) : 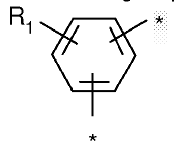 avec R1 étant un radical alkyle C₁ à C₄ linéaire ou ramifié, et les * symbolisant les points de rattachement du groupement A à chacun des deux atomes d'azote du reste du composé de formule générale (I), et
- R et R', identiques ou différents, sont choisis parmi :
- i) les radicaux de formule (III) : 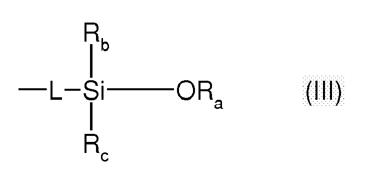
dans laquelle :
- L est une liaison simple ou un radical divalent carboné, notamment hydrocarboné, linéaire, ramifié et/ou cyclique, saturé ou insaturé, comprenant 1 à 18 atomes de carbone, et pouvant comprendre 1 à 4 hétéroatomes choisis parmi N, O et S;
- Ra est :
a) un radical carboné, notamment hydrocarboné, linéaire, ramifié et/ou cyclique, saturé ou insaturé, comprenant 1 à 18 atomes de carbone, et pouvant comprendre 1 à 8 hétéroatomes choisis parmi N, O, Si et S; ou bien
b) un radical siliconé de formule : 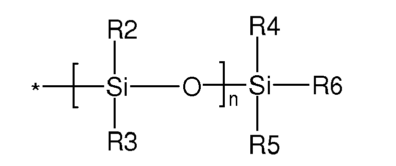 avec n étant compris entre 0 et 100;
et R2 à R6 étant, indépendamment les uns des autres, des radicaux carbonés, notamment hydrocarbonés linéaires ou ramifiés, ayant 1 à 12 atomes de carbone, et pouvant comprendre 1 à 4 hétéroatomes;
- Rb et Rc sont, indépendamment l'un de l'autre, choisis parmi:
a) les radicaux carbonés, notamment hydrocarbonés, linéaires, ramifiés et/ou cycliques, saturés ou insaturés, comprenant 1 à 18 atomes de carbone, et pouvant comprendre 1 à 4 hétéroatomes choisis parmi N, O, Si et S;
b) les radicaux de formule : 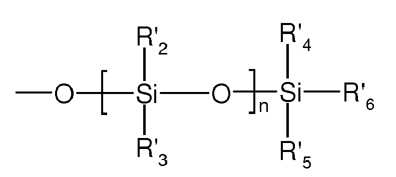 avec n étant compris entre 0 et 100;
et R'2 à R'6 étant, indépendamment les uns des autres, des radicaux carbonés, notamment hydrocarbonés linéaires ou ramifiés, ayant 1 à 12 atomes de carbone, et pouvant comprendre 1 à 4 hétéroatomes;
et
- ii) les radicaux alkyle en C₁ à C₃₀, linéaire, ramifié et/ou cyclique, saturé ou insaturé, et comprenant éventuellement 1 à 3 hétéroatomes choisis parmi O, S, FetN;
étant entendu que l'un au moins des radicaux R et/ou R' est de formule (III).

2. Composition selon la revendication 1, dans laquelle le groupement A est de formule : 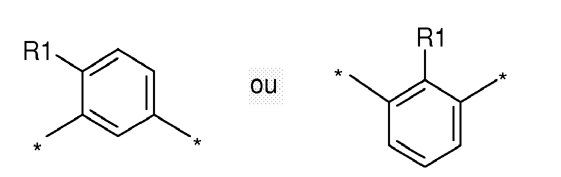 avec R1 et les * étant tels que définis dans la revendication 1.

3. Composition selon l'une des revendications précédentes, dans laquelle le groupement A est de formule :

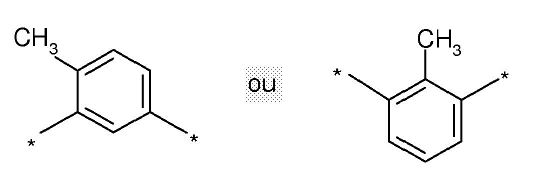

4. Composition selon l'une des revendications précédentes, dans laquelle L est de structure -(CH₂)n- avec n= 1 à 18; ou de structure -CH₂-CH(CH₃)-.

5. Composition selon l'une des revendications précédentes, dans laquelle le radical Ra est choisi parmi les radicaux :
- de structure -(CH₂)n'-CH₃ avec n'= 0 à 17;
- de structure -(CH₂)x-O-(CH₂)z-CH₃ ou bien -(CH₂)x-O-(CH₂)y-O-(CH₂)z-CH₃, avec x = 1 à 10; y=1 à 10 et z= 0 à 10; ou bien
- de structure -SiR₄R₅R₆, dans laquelle R4, R5 et R6 sont, indépendamment les uns des autres, des radicaux alkyle ayant 1 à 12 atomes de carbone; ou bien
- de formule : 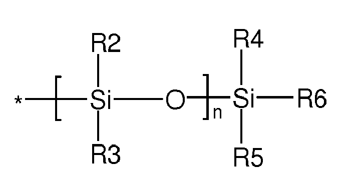
dans laquelle R2 à R6 sont, indépendamment les uns des autres, des radicaux alkyle ayant 1 à 12 atomes de carbone.

6. Composition selon l'une des revendications précédentes, dans laquelle Ra est de structure : 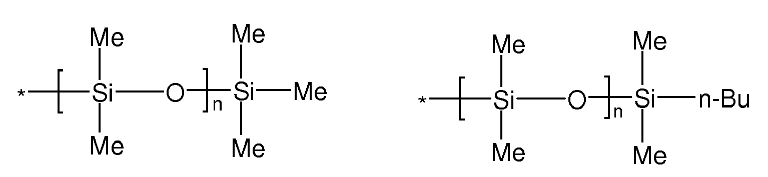 avec n=1 à 100; et encore plus particulièrement un radical :

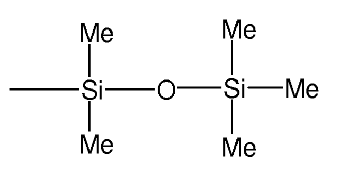

7. Composition selon l'une des revendications précédentes, dans laquelle les radicaux Rb et Rc, identiques ou différents, sont choisis parmi les radicaux :
- de structure -(CH₂)m-CH₃ avec m= 0 à 17; ou bien
- de structure -O-(CH₂)m'-CH₃ avec m'= 0 à 5; ou bien
- de structure -O-(CH₂)x-O-(CH₂)z-CH₃ ou -O-(CH₂)x-O-(CH₂)y-O-(CH₂)z-CH₃, avec x = 1 à 10; y=1 à 10 et z= 0 à 10; ou bien
- de structure : 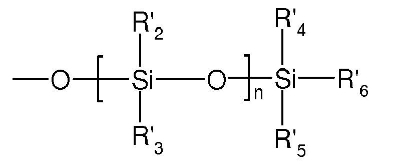 avec n étant compris entre 0 et 100; et R'2 à R'6 étant, indépendamment les uns des autres, des radicaux alkyle ayant 1 à 12 atomes de carbone.

8. Composition selon l'une des revendications précédentes, dans laquelle l'un au moins des radicaux R et/ou R' est choisi parmi les radicaux suivants : 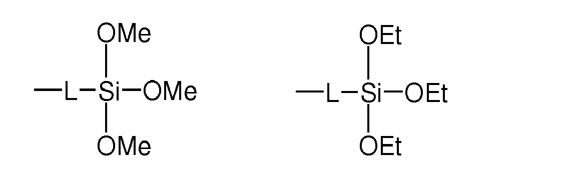 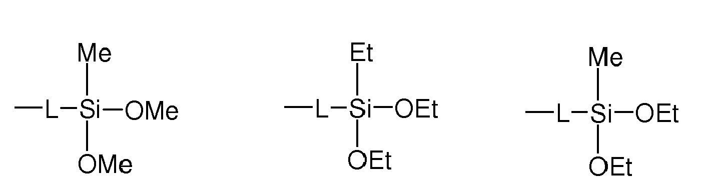 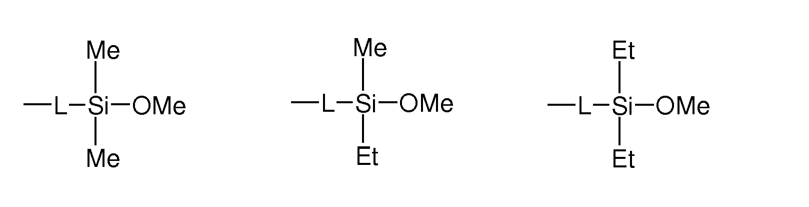 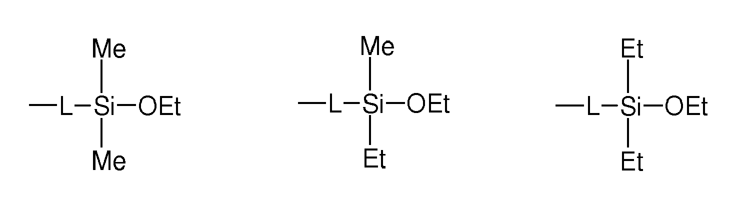 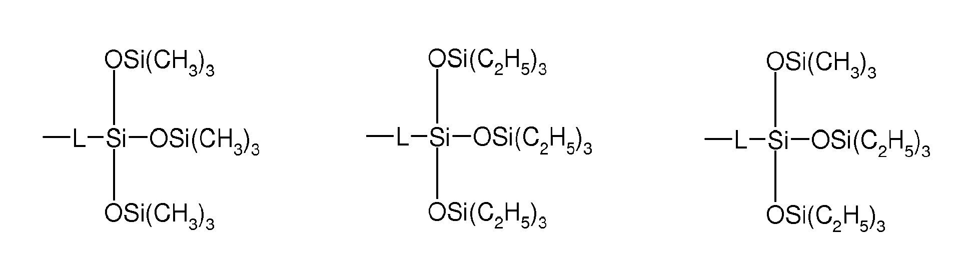 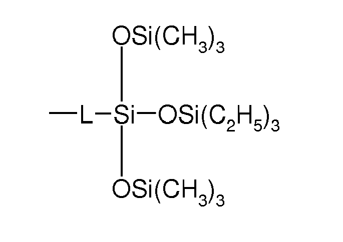 et également ceux de formule : 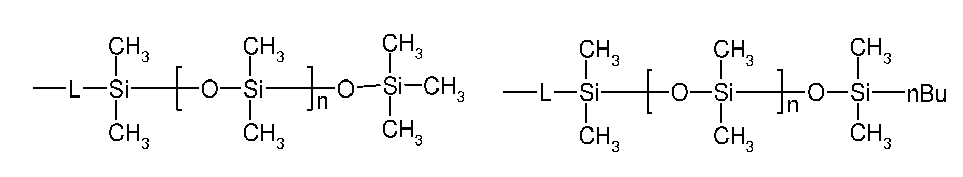 avec n variant de 0 à 100 et en particulier 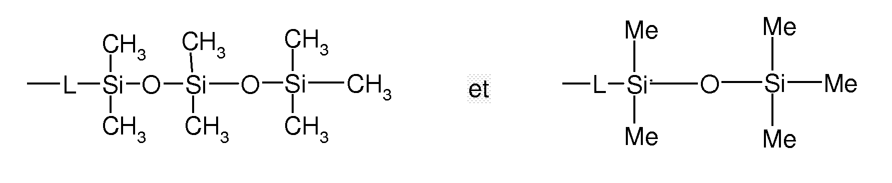 et 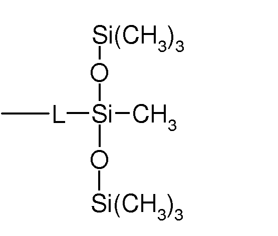 ou encore 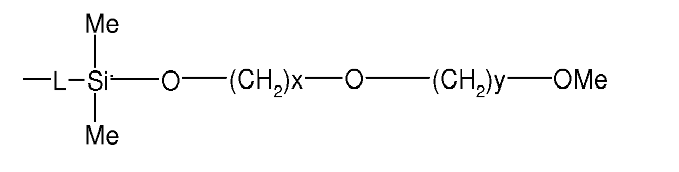 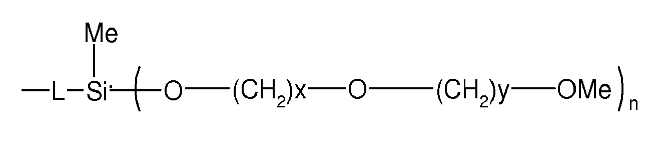 avec n=2 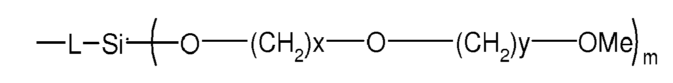 dans lesquelles x = 1 à 10; et y = 1 à 10; et L étant tel que défini ci-dessus.

9. Composition selon la revendication 8, dans laquelle L est un radical alkylène en C1-C8, linéaire ou ramifié, notamment méthylène, éthylène, propylène, butylène et notamment n-butylène, octylène ou de formule -CH₂-CH(CH₃)-.

10. Composition selon l'une des revendications précédentes, dans laquelle R et R', identiques ou différents, sont tous les deux de formule (III).

11. Composition selon l'une des revendications 1 à 9, dans laquelle l'un des radicaux R ou R' représente un radical alkyle en C₁ à C₃₀, linéaire, ramifié et/ou cyclique, saturé ou insaturé, et comprenant éventuellement 1 à 3 hétéroatomes choisis parmi O, S, F et N.

12. Composition selon la revendication 11, dans laquelle le radical R ou R' est choisi parmi : 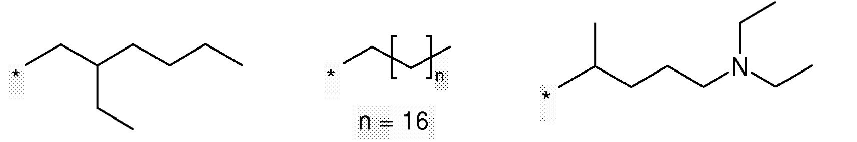 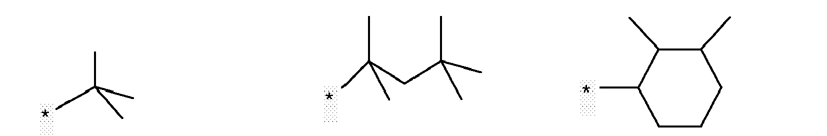 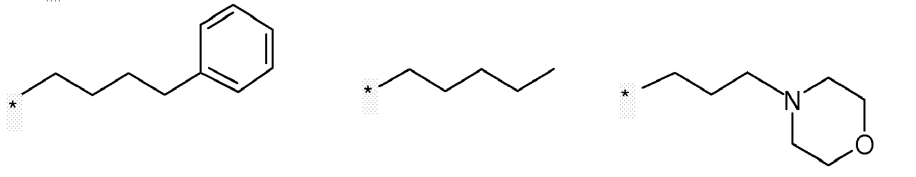 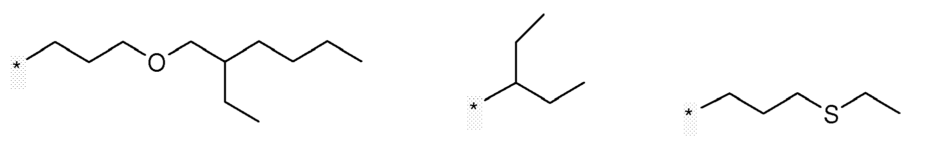 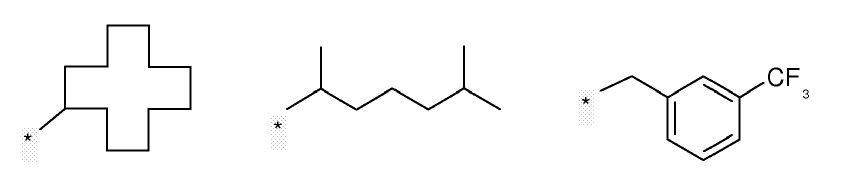 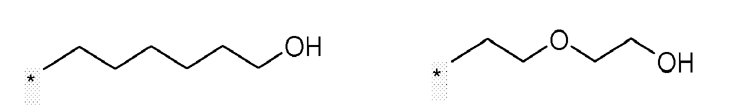 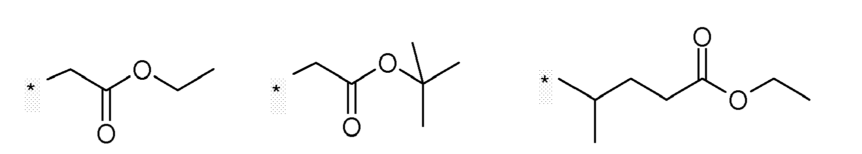 avec * ayant la définition donnée ci-dessus.

13. Composition selon l'une des revendications 1 à 9, dans laquelle le radical R ou R' représente un radical alkyle ramifié, notamment monoramifié, de préférence non cyclique, saturé ou insaturé, comprenant 3 à 16 atomes de carbone, et comprenant éventuellement 1 à 3 hétéroatomes choisis parmi O, S, F et/ou N.

14. Composition selon la revendication 13, dans laquelle le radical R ou R' est choisi parmi les radicaux tertio-butyle, 2-éthylhexyle ou de formule :

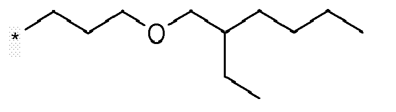

15. Composition selon l'une des revendications précédentes, dans laquelle le composé de formule (1) est choisi parmi, seul ou en mélange, les composés suivants, ainsi que leurs sels et isomères :

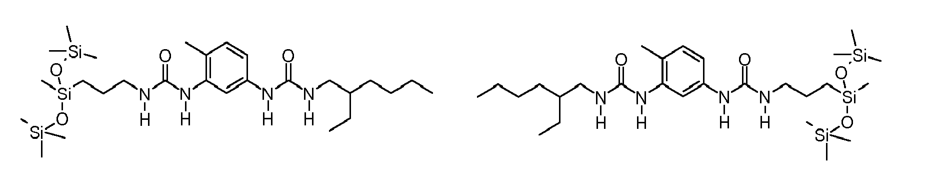

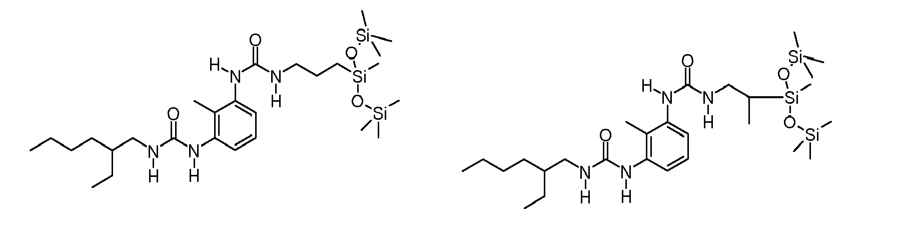

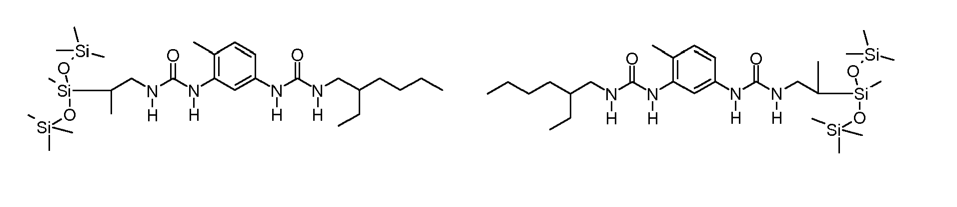

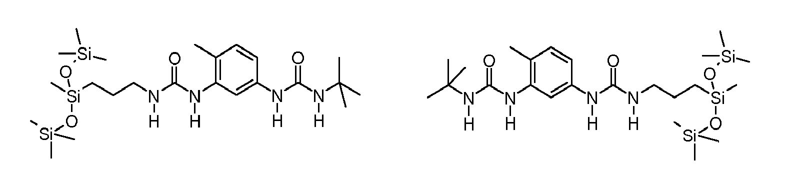

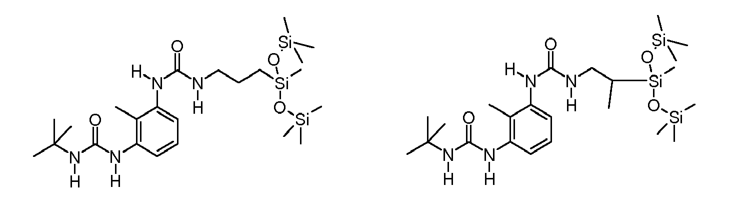

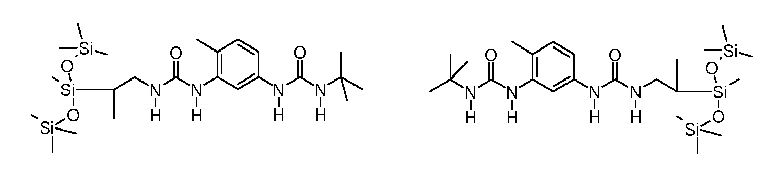

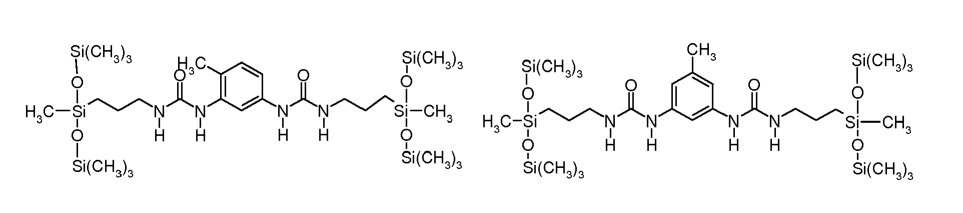

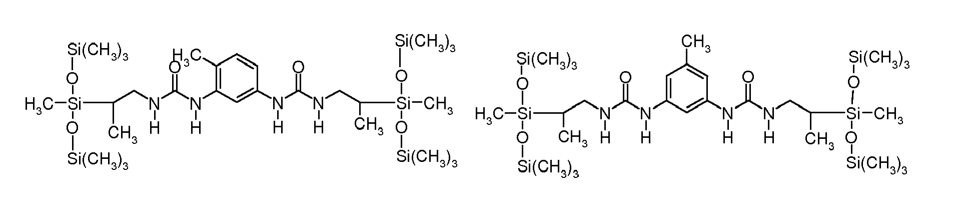

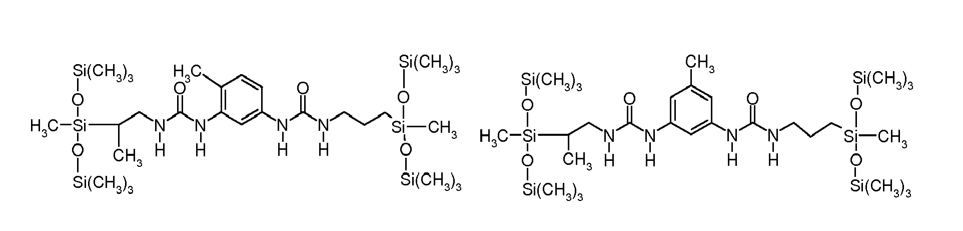

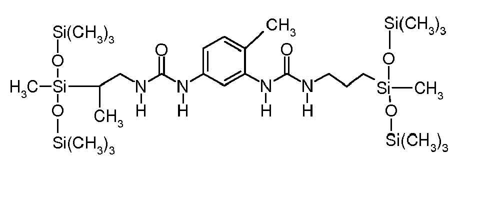

16. Composition selon l'une des revendications précédentes, comprenant de 0,01 à 20% en poids, notamment de 0,05 à 15% en poids, voire de 0,1 à 10% en poids, mieux de 1 à 8% en poids, et encore mieux de 2 à 5% en poids, de composé(s) de formule (1) par rapport au poids total de la composition.

17. Composition selon l'une des revendications précédentes, dans laquelle la phase grasse liquide comprend un ou plusieurs composé lipophiles choisis parmi les huiles siliconées, les huiles carbonées et les solvants carbonés, liquides à température ambiante.

18. Composition selon l'une des revendications précédentes, dans laquelle la phase grasse liquide comprend au moins un composé lipophile choisi parmi, seul ou en mélange :
1/ les esters des acides monocarboxyliques avec les monoalcools et polyalcools; notamment de formule R'₁-CO-O-R'₂ où :
R'₁ représente un radical alkyle linéaire ou ramifié de 1 à 40 atomes de carbone, comprenant éventuellement une ou plusieurs double liaisons éthyléniques, éventuellement substitué et dont la chaîne hydrocarbonée peut être interrompue par un ou plusieurs hétéroatomes choisis parmi N et O et/ou une ou plusieurs fonctions carbonyles, et
R'₂ représente un radical alkyle linéaire ou ramifié de 1 à 40 atomes de carbone, comprenant éventuellement une ou plusieurs double liaisons éthyléniques, éventuellement substitué et dont la chaîne hydrocarbonée peut être interrompue par un ou plusieurs hétéroatomes choisis parmi N et O et/ou une ou plusieurs fonctions carbonyles.
2/ les huiles fluorées;
3/ les huiles végétales hydrocarbonées à forte teneur en triglycérides constitués d'esters d'acides gras et de glycérol dont les acides gras peuvent avoir des longueurs de chaînes variées, ces dernières pouvant être linéaires ou ramifiées, saturées ou insaturées;
4/ les éthers en C₆ à C₄₀,
5/ les acides gras en C₈-C₃₂,
6/ les alcools dits 'gras', et notamment les monoalcools, en C6-C32;
7/ les hydrocarbures ou fluorocarbures, linéaires ou ramifiés, d'origine synthétique ou minérale;
8/ les huiles bifonctionnelles, comprenant deux fonctions choisies parmi ester et/ou amide et comprenant de 6 à 30 atomes de carbone, notamment 8 à 28 atomes de carbone, mieux de 10 à 24 carbones, et 4 hétéroatomes choisis parmi O et N; de préférence les fonctions amide et ester étant dans la chaîne;
9/ les huiles siliconées volatiles ou non volatiles, éventuellement fluorées, linéaires ou cycliques.

19. Composition selon l'une des revendications précédentes, dans laquelle la phase grasse liquide comprend au moins un composé lipophile choisi parmi, seul ou en mélange :
- les esters en C₈-C₄₈, éventuellement incorporant dans leur chaîne hydrocarbonée un ou plusieurs hétéroatomes parmi N et O et/ou une ou plusieurs fonctions carbonyle; et plus particulièrement l'huile de purcellin (octanoate de cétostéaryle), l'isononanoate d'isononyle, le myristate d'isopropyle, le palmitate d'éthyl-2-hexyle, le stéarate d'octyl-2-dodécyle, l'érucate d'octyl-2-dodécyle, le l'isostéarate d'isostéaryle, et les heptanoates, octanoates, décanoates ou ricinoléates d'alcools ou de polyalcools, par exemple d'alcools gras, ainsi que le N-lauroyl sarcosinate d'isopropyle;
- les perfluoropolyéthers, les perfluoroalcanes comme la perfluorodécaline, les perfluorodamantanes, les monoesters, diesters et triesters de perfluoroalkylphosphates et les huiles esters fluorés;
- les huiles de germe de blé, de maïs, de tournesol, de karité, de ricin, d'amandes douces, de macadamia, d'abricot, de soja, de colza, de coton, de luzerne, de pavot, de potimarron, de sésame, de courge, d'avocat, de noisette, de pépins de raisin ou de cassis, d'onagre, de millet, d'orge, de quinoa, d'olive, de seigle, de carthame, de bancoulier, de passiflore, de rosier muscat ; ou encore les triglycérides des acides caprylique/caprique;
- les acides oléique, linoléique ou linolénique,
- l'alcool oléïque et l'octyldodécanol;
- les huiles de paraffine (telles que les isoparaffines en C₈-C₁₆, l'isododécane, l'isohexadécane) et leurs dérivés, la vaseline, les polydécènes, les polyisobutènes hydrogénés tel que le squalane, et leurs mélanges;
- les alcanes, linéaires ou ramifiés, en C₆-C₄₈, notamment volatiles;
- les polydiméthylsiloxanes (PDMS) volatiles, linéaires ou cycliques ayant de 3 à 7 atomes de silicium, et notamment l'octyltriméthicone, l'hexyltriméthicone, la décaméthylcyclopentasiloxane (ou D5), l'octaméthylcyclotétrasiloxane (ou D4), la dodécaméthylcyclohexasiloxane (ou D6), la décaméthyltétrasiloxane (ou L4), l'heptaméthyloctyltrisiloxane, le dodécaméthylpentasiloxane, le polyméthylcétyldiméthylsiloxane; les huiles siliconées fluorées telles que les silicones à groupes alkyle et perfluoralkyle, les silicones à groupes latéraux oxyéthylénés/oxypropylénés (OE/PP) et à groupes perfluorés, les silicones à groupes latéraux perfluorés ou polyfluorés et à groupes latéraux glycérolés, et les perfluoroalkylméthylphénylsiloxanes;
- les huiles siliconées non volatiles tels que les polydiméthylsiloxanes (PDMS), des polyalkylméthylsiloxanes, des diméthicone copolyols, des alkylméthicone copolyols, la cétyldiméthicone, des silicones à groupes alkylglycéryl éthers, des silicones à groupes amines latéraux et le dilauroyltriméthylol propane siloxysilicate; les polydiméthylsiloxanes comportant des groupements alkyle, alcoxy ou phényle, pendants et/ou en bout de chaîne siliconée, groupements ayant chacun de 2 à 24 atomes de carbone; les silicones phénylées comme les phényl triméthicones, les phényl diméthicones, les phényl triméthylsiloxy diphénylsiloxanes, les diphényl diméthicones, les diphényl méthyldiphényl trisiloxanes, les 2-phényléthyl triméthylsiloxysilicates, la cétyldiméthicone, les silicones à groupes alkylglycéryl éthers, les silicones à groupe amines latéraux, les silicones fluorées à groupement(s) pendant(s) ou en bout de chaîne ayant de 1 à 12 atomes de carbone dont tout ou partie des atomes d'hydrogène est substitué par des atomes de fluor, les diméthiconols et/ou leurs mélanges.

20. Composition selon l'une des revendications précédentes, dans laquelle la phase grasse liquide comprend au moins un composé lipophile choisi parmi :
- les monoalcools en C₆-C₃₂, de préférence en C₈-C₂₈, mieux en C₁₂-C₂₆ et préférentiellement l'octyldodécanol;
- les alcanes ramifiés en C₆-C₃₂, de préférence en C₈-C₂₈, mieux en C₁₂-C₂₆ et préférentiellement l'isododécane ou le parléam de formule -(CH₂-CH(CH₃))ₙ-avec n étant un entier variant de 4 à 8;
- les alcanes linéaires en C₁₃-C₄₈, de préférence en C₁₈-C₄₀, mieux en C₂₀-C₃₂,
- les huiles bifonctionnelles, comprenant deux fonctions choisies parmi ester et/ou amide et comprenant de 6 à 30 atomes de carbone, notamment 8 à 28 atomes de carbone, mieux de 10 à 24 carbones, et 4 hétéroatomes choisis parmi O et N; de préférence les fonctions amide et ester étant dans la chaîne; en particulier l'huile bifonctionnelle peut être le N-lauroylsarcosinate d'isopropyle de formule suivante : 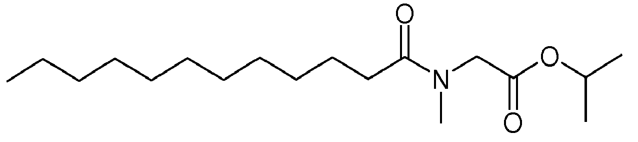
- les esters en C₈-C₄₈, éventuellement incorporant dans leur chaîne hydrocarbonée un ou plusieurs hétéroatomes parmi N et O et/ou une ou plusieurs fonctions carbonyle; et plus particulièrement l'huile de purcellin (octanoate de cétostéaryle), l'isononanoate d'isononyle, le myristate d'isopropyle, le palmitate d'éthyl-2-hexyle, le stéarate d'octyl-2-dodécyle, l'érucate d'octyl-2-dodécyle, le l'isostéarate d'isostéaryle, et les heptanoates, octanoates, décanoates ou ricinoléates d'alcools ou de polyalcools, par exemple d'alcools gras;
- les huiles siliconées, volatiles ou non, et notamment les polydiméthylsiloxanes (PDMS) linéaires ou cycliques ayant de 3 à 7 atomes de silicium tels que la décaméthylcyclopentasiloxane, l'octaméthylcyclotétrasiloxane, la dodécaméthylcyclohexasiloxane et la décaméthyltétrasiloxane; et les silicones phénylées comme les phényl triméthicones;
- leurs mélanges.

21. Composition selon l'une des revendications 17 à 20, dans laquelle les composés lipophiles représentent 20 à 100%, voire de 40 à 99 %, notamment de 60 à 95% en poids de la phase grasse liquide totale.

22. Composition selon l'une des revendications précédentes, comprenant en outre au moins un constituant choisi parmi les cires, les composés pâteux, l'eau, les solvants organiques miscibles à l'eau; les matières colorantes, organique ou inorganique; les charges, les vitamines, les épaississants, les gélifiants, les oligo-éléments, les adoucissants, les séquestrants, les parfums, les agents alcalinisants ou acidifiants, les conservateurs, les filtres solaires, les tensioactifs, les anti-oxydants, les agents anti-chutes des cheveux, les agents anti-pelliculaires, les agents propulseurs, les céramides, les polymères, les actifs cosmétiques, et leurs mélanges.

23. Composition selon l'une des revendications précédentes, se présentant sous forme d'une composition de maquillage, notamment un produit pour le teint tel qu'un fond de teint, un fard à joues ou à paupières; un produit pour les lèvres tel qu'un rouge à lèvres ou un soin des lèvres; un produit anti-cernes; un blush, un mascara, un eye-liner; un produit de maquillage des sourcils, un crayon à lèvres ou à yeux; un produit pour les ongles tel qu'un vernis à ongles ou un soin des ongles; un produit de maquillage du corps ou des cheveux (mascara ou laque pour cheveux); d'une composition de protection ou de soin de la peau du visage, du cou, des mains ou du corps, notamment une composition anti-rides, anti-fatigue, anti-âge, permettant de donner un coup d'éclat à la peau, une composition hydratante ou traitante; une composition anti-solaire ou après-soleil ou de bronzage artificiel; d'une composition capillaire, notamment pour la coloration, le soin, l'hygiène des cheveux, le coiffage, le maintien de la coiffure ou la mise en forme des cheveux.

24. Procédé de traitement cosmétique, notamment de maquillage, de nettoyage, de protection solaire, de mise en forme, de coloration et/ou de soin, des matières kératiniques, notamment de la peau du corps ou du visage, des lèvres, des ongles, des sourcils, des cheveux et/ou des cils, comprenant l'application sur lesdites matières d'une composition cosmétique telle que définie à l'une des revendications précédentes.

25. Utilisation d'un composé de formule (I) tel que défini dans l'une des revendications 1 à 15, pour texturer une composition cosmétique ou pharmaceutique comprenant, dans un milieu physiologiquement acceptable, une phase grasse.

26. Composé de formule générale (la), ou l'un de ses sels et/ou isomères : 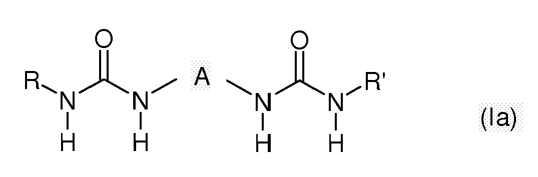 dans laquelle :
- A est un groupement de formule (II) : 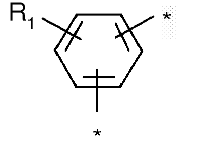 avec R1 étant un radical alkyle C₁ à C₄ linéaire ou ramifié, et les * symbolisant les points de rattachement du groupement A à chacun des deux atomes d'azote du reste du composé de formule générale (I), et
- R et R', identiques ou différents, sont choisis parmi :
- i) les radicaux de formule (III) : 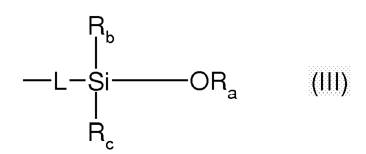
dans laquelle :
- L est une liaison simple ou un radical divalent carboné, notamment hydrocarboné, linéaire, ramifié et/ou cyclique, saturé ou insaturé, comprenant 1 à 18 atomes de carbone, et pouvant comprendre 1 à 4 hétéroatomes choisis parmi N, O et S;
- Ra est :
a) un radical carboné, notamment hydrocarboné, linéaire, ramifié et/ou cyclique, saturé ou insaturé, comprenant 1 à 18 atomes de carbone, et pouvant comprendre 1 à 8 hétéroatomes choisis parmi N, O, Si et S; ou bien
b) un radical siliconé de formule : 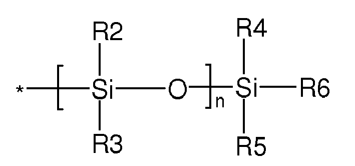 avec n étant compris entre 0 et 100; et R2 à R6 étant, indépendamment les uns des autres, des radicaux carbonés, notamment hydrocarbonés linéaires ou ramifiés, ayant 1 à 12 atomes de carbone, et pouvant comprendre 1 à 4 hétéroatomes, notamment O;
- Rb et Rc sont, indépendamment l'un de l'autre, choisis parmi:
a) les radicaux carbonés, notamment hydrocarbonés, linéaires, ramifiés et/ou cycliques, saturés ou insaturés, comprenant 1 à 18 atomes de carbone, et pouvant comprendre 1 à 4 hétéroatomes choisis parmi N, O, Si et S;
b) les radicaux de formule : 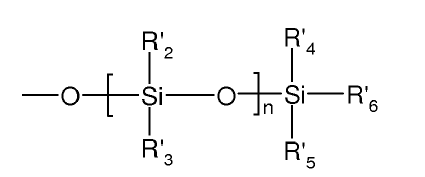 avec n étant compris entre 0 et 100; et R'2 à R'6 étant, indépendamment les uns des autres, des radicaux carbonés, notamment hydrocarbonés linéaires ou ramifiés, ayant 1 à 12 atomes de carbone, et pouvant comprendre 1 à 4 hétéroatomes, notamment O;
et
- ii) les radicaux alkyle ramifiés, saturés ou insaturés, comprenant 3 à 16 atomes de carbone, et comprenant éventuellement 1 à 3 hétéroatomes choisis parmi O, S, F et/ou N;
étant entendu que l'un au moins des radicaux R et/ou R' est de formule (III).

27. Composé selon la revendication 26, dans lequel le radical R ou R' est choisi parmi les radicaux tertio-butyle, 2-éthylhexyle ou de formule :

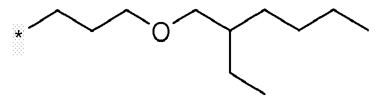

28. Composé selon l'une des revendications 26 à 27, choisi parmi, seul ou en mélange, les composés suivants, ainsi que leurs sels et isomères :
